# EUROPÄISCHE PATENTSCHRIFT

(11) **EP 1 663 932 B1**
(45) Veröffentlichungstag und Bekanntmachung des Hinweises auf die Patenterteilung: **19.03.2008**
(21) Anmeldenummer: 04786993.8
(22) Anmeldetag: 23.09.2004
(51) Int. Cl.: C07C 45/28

(54) **VERFAHREN ZUR HERSTELLUNG VON CYCLODODECANON**
METHOD FOR PRODUCING CYCLODODECANONE
PROCEDE DE PRODUCTION DE CYCLODODECANONE

(30) Priorität: 25.09.2003 DE 10344594
(43) Veröffentlichungstag der Anmeldung: 07.06.2006
(73) Patentinhaber: BASF SE, 67056 Ludwigshafen (DE)
(72) Erfinder: TELES, Joaquim, 67166 Otterstadt (DE); RÖßLER, Beatrice, 67098 Bad Dürkheim (DE); PINKOS, Rolf, 67098 Bad Dürkheim (DE); GENGER, Thomas, 67245 Lambsheim (DE); PREISS, Thomas, 67256 Weisenheim am Sand (DE)
(74) Vertreter: Isenbruck, Günter
(86) Internationale Anmeldenummer: PCT/EP2004/010680
(87) Internationale Veröffentlichungsnummer: WO 2005/030689

(56) Entgegenhaltungen:
- DE-A1- 19 856 862
- US-A- 2 636 898
- US-A- 5 321 176
- DATABASE CAPLUS [Online] CHEMICAL ABSTRACTS SERVICE, COLUMBUS, OHIO, US; PANOV, GENNADY IVANOVICH ET AL: "Method for producing monocyclic ketones C7-12" XP002321385 gefunden im STN Database accession no. 2003:757660 & WO 03/078375 A1 (INSTITUT KATALIZA IMENI G. K. BORESKOVA SIBIRSKOGO OTDELENIYA ROSSIISK) 25. September 2003 (2003-09-25)
- E.V. STAROKON ET AL.: "Liquid phase oxidation of alkenes with nitrous oxide to carbonyl compounds" ADVANCED SYNTHESIS & CATALYSIS , 346(2+3), 268-274 CODEN: ASCAF7; ISSN: 1615-4150, 2004, XP002321384 in der Anmeldung erwähnt
- G.I. PANOV ET AL.: "Non-catalytic liquid phase oxidation of alkenes with nitrous oxide. 1. Oxidation of cyclohexene to cyclohexanone" REACT. KINET. CATAL. LETT., Bd. 76, Nr. 2, 2002, Seiten 401-406, XP002321392 in der Anmeldung erwähnt
- A.K. URIARTE: "Nitrous oxide (N2O) - Waste to value" STUDIES IN SURFACE SCIENCE AND CATALYSIS, Bd. 130, 2000, Seiten 743-748, XP009045195 in der Anmeldung erwähnt

## Beschreibung

Die vorliegende Erfindung betrifft ein Verfahren zur Herstellung von Cyclododecanon aus Cyclododecen, wobei Cyclododecen durch Umsetzung mit Distickstoffmonoxid zu Cyclododecanon oxidiert wird wobei als Distickstoffmonoxidquelle mindestens ein Distickstoffmonoxid enthaltendes Abgas mindestens eines industriellen Verfahrens dient. In einer bevorzugten Ausführungsform betrifft die vorliegende Erfindung ein zweistufiges Verfahren, wobei Cyclododecatrien durch Partialhydrierung zu Cyclododecen umgesetzt wird und das resultierende Cyclododecen durch Umsetzung mit Distickstoffmonoxid zu Cyclododecanon oxidiert wird.

Cyclododecanon ist ein wichtiges Zwischenprodukt für die Herstellung von beispielsweise Laurinlactam, Dodecandicarbonsäure und daraus abgeleiteten Polyamiden wie beispielsweise Nylon 12 oder Nylon 6.12.

Cyclododecanon wird gemäß des gängigen technischen Verfahrens durch Luftoxidation von Cyclododecan in Anwesenheit von Borsäure zu Cyclododecylborat, Hydrolyse des Borates zu Cyclododecanol und anschließende Dehydrierung des Cyclododecanols hergestellt. Cyclododecan selbst wird weiterhin durch vollständige Hydrierung von Cyclododecatrien (CDT) gewonnen. Eine Beschreibung dieses technischen Verfahrens zur Synthese von Cyclododecanon findet sich unter anderem in T. Schiffer, G. Oenbrink, "Cyclododecanol, Cyclododecanon and Laurofactam" in Ullmann's Encyclopedia of Industrial Chemistry, 6th Edition, 2000, Electronic Release, Wiley VCH.

Das genannte technische Verfahren weist indessen eine Reihe von Nachteilen auf.

Zum einen gewährleistet die Oxidation von Cyclododecan mit Sauerstoff nur bei geringen Umsätzen eine akzeptable Selektivität. Auch unter Zusatz von Borsäure, die das entstandene Cyclododecanol in Form von Borsäureester vor weiterer Oxidation schützt, darf der Cyclododecan-Umsatz nicht über 30% liegen. Nach der Oxidation müssen die Borsäureester in einem getrennten Schritt hydrolysiert werden, und sowohl die Borsäure als auch das nicht umgesetzte Cyclododecan müssen in die Oxidation rückgeführt werden. Dabei entstehen auch Bor-haltige Abfälle, die schwer zu entsorgen sind. Als Hauptprodukte bilden sich dabei Cyclododecanol und Cyclododecanon im Verhältnis 10:1.

Zum anderen müssen das gebildete Gemisch aus Cyclododecanol und Cyclododecanon destillativ aufgetrennt und das Cyclododecanol durch Dehydrierung in Cyclododecanon überführt werden. Diese Dehydrierung ist endotherm und liefert ebenfalls nur einen partiellen Umsatz. Das nicht umgesetzte Cyclododecanol muss dann wiederum destillativ abgetrennt und in das Verfahren zurückgeführt werden.

Als Folge des nicht vollständigen Umsatzes beinhaltet das konventionelle Verfahren mehrere große Rückführströme und eine Reihe von technisch aufwändigen destillativen Trennungen.

Eine der der vorliegenden Erfindung zugrunde liegenden Aufgaben war es daher, ein neues Verfahren zur Herstellung von Cyclododecanon bereitzustellen. Diese Aufgabe wurde gelöst durch ein Verfahren, in dem Cyclododecen mit Distickstoffmonoxid als Oxidationsmittel zu Cyclododecanon umgesetzt wird wobei als Distickstoffmonoxidquelle mindestens ein Distickstoffmonoxid enthaltendes Abgas mindestens eines industriellen Verfahrens dient. Insbesondere wurde diese Aufgabe gelöst durch ein Verfahren, bei dem in einem Schritt Cyclododecen durch Partialhydrierung aus Cyclododecatrien hergestellt wird und in einem weiteren Schritt Cyclododecen mit Distickstoffmonoxid als Oxidationsmittel zu Cyclododecanon umgesetzt wird wobei als Distickstoffmonoxidquelle mindestens ein Distickstoffmonoxid enthaltendes Abgas mindestens eines industriellen Verfahrens dient. In den erfindungsgemäßen Verfahren können reines Cyclododecen oder ein Gemisch, enthaltend Cyclododecen, und reines Distickstoffmonoxid oder ein Gemisch, enthaltend Distickstoffmonoxid, eingesetzt werden. Weiterhin kann Cyclododecen als cis-Isomer oder als trans-Isomer oder als Mischung aus cis- und trans-Isomer vorliegen.

Die Oxidation einer olefinischen Verbindung zu einem Aldehyd oder einem Keton mittels Distickstoffmonoxid ist beispielsweise beschrieben in der GB 649,680 oder der dazu äquivalenten US 2,636,898. Als cyclische olefinische Verbindungen werden dort allerdings lediglich Cyclopenten, Cyclohexen, und Cycloocten beschrieben. In beiden Schriften wird ganz allgemein offenbart, dass die Oxidation prinzipiell in Anwesenheit eines geeigneten Oxidationskatalysators erfolgen kann.

WO 03/078375 offenbart ein Verfahren zur Herstellung monocyclischer C₇-C₂₀-Ketone durch Kontaktieren von Distickstoffmonoxid mit flüssigen monocyclischen Alkenen der Formel CₙH₂ₙ₋₂, die 7 bis 20 Kohlenstoffe im Ring enthalten. Das Verfahren wird bei einer Temperatur zwischen 20 und 350°C und einem Distickstoffmonoxid-Druck im Bereich von 0,01 bis 100 atm durchgeführt.

In den neueren wissenschaftlichen Artikeln von G. L Panov et al., "Non-Catalytic Liquid Phase Oxidation of Alkenes with Nitrous Oxide. 1. Oxidation of Cyclohexene to Cyclohexanone", React. Kinet. Catal. Lett. Vol. 76, No. 2 (2002) S. 401-405, und K. A. Dubkov et al., "Non-Catalytic Liquid Phase Oxidation of Alkenes with Nitrous Oxide. 2. Oxidation of Cyclopentene to Cyclopentanone", React. Kinet. Catal. Lett. Vol. 77, No. 1 (2002) S. 197-205 werden ebenfalls Oxidationen von olefinischen Verbindungen mit Distickstoffmonoxid beschrieben. Allerdings beschränken sich die diesbezüglichen Offenbarungen ausschließlich auf Cyclopenten und Cyclohexen.

Auch ein wissenschaftlicher Artikel "Liquid Phase Oxidation of Alkenes with Nitrous Oxide to Carbonyl Compounds" von E. V. Starokon et al. in Adv. Synth. Catal. 2004, 346, 268 - 274 beinhaltet eine mechanistische Studie der Oxidation von Alkenen mit Distickstoffmonoxid in flüssiger Phase.

Demgemäß betrifft die vorliegende Erfindung ein Verfahren zur Herstellung von Cyclododecanon durch Umsetzung von Cyclododecen mit Distickstoffmonoxid, wobei als Distickstoffmonoxidquelle mindestens ein Distickstoffmonoxid enthaltendes Abgas mindestens eines industriellen Verfahrens dient.

Das zur Umsetzung verwendete Distickstoffmonoxid kann grundsätzlich in reiner Form oder in Form eines geeigneten Gasgemisches, enthaltend Distickstoffmonoxid, eingesetzt werden. Dabei kann das Distickstoffmonoxid weiterhin grundsätzlich aus jeder beliebigen Quelle stammen.

Der Begriff "Gasgemisch", wie er im Rahmen der vorliegenden Erfindung verwendet wird, bezeichnet ein Gemisch aus zwei oder mehr Verbindungen, die sich bei Umgebungsdruck und Umgebungstemperatur im gasförmigen Zustand befinden. Bei veränderter Temperatur oder verändertem Druck kann das Gasgemisch auch in einem anderen Aggregatzustand vorliegen, beispielsweise flüssig oder überkritisch, vorzugsweise flüssig, und wird im Rahmen der vorliegenden Erfindung weiter als Gasgemisch bezeichnet.

Wird ein Gasgemisch eingesetzt, so ist dessen Gehalt an Distickstoffmonoxid im Wesentlichen beliebig, solange gewährleistet ist, dass die erfindungsgemäße Umsetzung möglich ist.

Gemäß einer bevorzugten Ausführungsform des erfindungsgemäßen Verfahrens wird ein mindestens 10 Vol.-% Distickstoffmonoxid enthaltendes Gasgemisch eingesetzt, wobei wiederum bevorzugt Gemische mit einem Distickstoffmonoxid-Gehalt im Bereich von 20 bis 99,9 Vol.-%, weiter bevorzugt im Bereich von 40 bis 99,5 Vol.-%, weiter bevorzugt im Bereich von 60 bis 99,5 Vol.-% und insbesondere bevorzugt im Bereich von 80 bis 99,5 Vol.-% eingesetzt werden.

Im Rahmen der vorliegenden Erfindung wird die Zusammensetzung der Gasgemische in Vol.-% angegeben. Dabei beziehen sich die Angaben auf die Zusammensetzung der Gasgemische bei Umgebungsdruck und Umgebungstemperatur.

Der Begriff "Gasgemisch", wie er im Rahmen der vorliegenden Erfindung verwendet wird, bezeichnet weiterhin Gemische, die neben Distickstoffmonoxid noch mindestens eine weitere Komponente, vorzugsweise ein weiteres Gas, enthalten. Dabei kann es sich bei der Komponente auch um ein Gas handeln, das bei den gewählten Bedingungen beispielsweise flüssig vorliegt. Hierbei sind im Wesentlichen sämtliche Gase denkbar, solange gewährleistet ist, dass die Umsetzung von Cyclododecen mit Distickstoffmonoxid möglich ist. Insbesondere sind demzufolge Gasgemische bevorzugt, die neben Distickstoffmonoxid mindestens ein Inertgas enthalten. Der Begriff "Inertgas", wie er im Rahmen der vorliegenden Erfindung verwendet wird, bezeichnet ein Gas, das sich hinsichtlich der Umsetzung von Distickstoffmonoxid mit Cyclododecen inert verhält. Als Inertgase sind beispielsweise Stickstoff, Kohlendioxid, Kohlenmonoxid, Wasserstoff, Wasser, Argon, Methan, Ethan und Propan zu nennen.

Ebenso können im Gasgemisch auch Komponenten, vorzugsweise Gase enthalten sein, die sich bei der Umsetzung von N₂O mit Cyclododecen nicht als Inerte, vorzugsweise Inertgase verhalten. Als solche Gase sind unter anderem NOₓ oder beispielsweise Sauerstoff zu nennen. Der Begriff "NOₓ'', wie er im Rahmen der vorliegenden Erfindung verwendet wird, bezeichnet sämtliche Verbindungen NₐO_{b} außer N₂O, wobei a 1 oder 2 ist und b eine Zahl von 1 bis 6. Statt dem Begriff "NOₓ" wird im Rahmen der vorliegenden Erfindung auch der Begriff "Stickoxide" verwendet. In einem solchen Fall ist es bevorzugt, solche Gasgemische einzusetzen, deren Gehalt an diesen Gasen im Bereich von 0 bis 0,5 Vol.-%, bezogen auf das Gesamtvolumen des Gasgemisches, liegt.

Demgemäß beschreibt die vorliegende Erfindung auch ein wie oben beschriebenes Verfahren, das dadurch gekennzeichnet ist, dass das Gasgemisch 0 bis 0,5 Vol-% Sauerstoff oder 0 bis 0,5 Vol% Stickoxide oder sowohl 0 bis 0,5 Vol% Sauerstoff als auch 0 bis 0,5 Vol-% Stickoxide, jeweils bezogen auf das Gesamtvolumen des Gasgemisches, enthält. Ein Wert von beispielsweise 0,5 Vol.% bezeichnet hierbei einen Gesamtgehalt aller möglichen Stickoxide außer N₂O von 0,5 Vol.-%.

Grundsätzlich kann die Zusammensetzung der Gemische im Rahmen der vorliegenden Erfindung auf jede dem Fachmann bekannte Weise bestimmt werden. Die Zusammensetzung der Gasgemische wird im Rahmen der vorliegenden Erfindung vorzugsweise gaschromatographisch bestimmt. Sie kann jedoch auch mittels UV-Spektroskopie, IR-Spektroskopie oder nasschemisch bestimmt werden.

Erfindungsgemäß kann Distickstoffmonoxid oder das Gasgemisch enthaltend Distickstoffmonoxid in jeder Form eingesetzt werden, insbesondere gasförmig oder auch in flüssiger Form. Dabei kann Distickstoffmonoxid oder das Gasgemisch enthaltend Distickstoffmonoxid mit allen dem Fachmann bekannten Verfahren verflüssigt werden, vorzugsweise durch geeignete Wahl des Drucks und der Temperatur.

Erfindungsgemäß ist es auch möglich, dass Distickstoffmonoxid oder das Gasgemisch enthaltend Distickstoffmonoxid zunächst in einem geeigneten Lösungsmittel absorbiert wird und so der Reaktion zugesetzt wird.

Gemäß der vorliegenden Erfindung ist die Distickstoffmonoxidquelle mindestens ein Distickstoffmonoxid enthaltendes Abgas eines chemischen Verfahrens. Im Rahmen der vorliegenden Erfindung sind auch Ausführungsformen umfasst, bei denen mindestens zwei Distickstoffmonoxid enthaltende Abgase einer einzigen Anlage als Distickstoffmonoxidquelle dienen. Ebenso sind Ausführungsformen umfasst, bei denen mindestens ein Distickstoffmonoxid enthaltendes Abgas einer Anlage und mindestens ein weiteres Distickstoffmonoxid enthaltendes Abgas mindestens einer weiteren Anlage als Distickstoffmonoxidquelle dienen.

Es ist auch möglich Distickstoffmonoxid gezielt herzustellen. Möglich ist unter anderem gezielt unter anderem die Herstellung über die thermische Zersetzung von NH₄NO₃, wie dies beispielsweise in der US 3,656,899 beschrieben ist, deren diesbezüglicher Inhalt durch Bezugnahme in den Kontext der vorliegenden Anmeldung vollumfänglich aufgenommen wird. Ebenfalls möglich ist die Herstellung über die katalytische Oxidation von Ammoniak, wie dies beispielsweise in der US 5,849,257 oder in der WO 98/25698 beschrieben ist.

Der Begriff "Distickstoffmonoxidquelle" bezeichnet im Rahmen der vorliegende Erfindung sowohl Ausführungsformen, in denen das genannte Abgas in unmodifizierter Form in der erfindungsgemäßen Umsetzung von Cyclododecen eingesetzt wird, als auch Ausführungsformen, in denen mindestens eines der genannten Abgase einer Modifikation unterworfen wird.

Der Begriff "Modifikation", wie er in diesem Zusammenhang im Rahmen der vorliegenden Erfindung verwendet wird, bezeichnet jedes geeignete Verfahren, mit dem die chemische Zusammensetzung eines Abgases verändert wird. Demgemäß umfasst der Begriff "Modifikation" unter anderem Ausführungsformen, in denen ein Distickstoffmonoxid enthaltendes Abgas bezüglich des Distickstoffmonoxidgehaltes gemäß mindestens eines geeigneten Verfahrens aufkonzentriert wird. Solche Verfahren sind beispielsweise in der DE-OS 27 32 267, der EP 1 076 217 A2 oder der WO 00/73202 A1 beschrieben.

Das Gasgemisch kann dabei im Rahmen der vorliegenden Erfindung auch einer Modifikation unterworfen werden, um die Konzentration von inerten und störenden Verbindungen im Gasgemisch zu reduzieren.

Diese Modifikation kann erfindungsgemäß beispielsweise eine Absorption des Gasgemisches in einem geeigneten Lösungsmittel und anschließende Desorption umfassen, um inerte Verbindungen aus dem Gasgemisch zu entfernen. Ein geeignetes Lösungsmittel für die Absorption ist beispielsweise Wasser, wie in der DT 20 40 219 beschrieben wird.

Erfindungsgemäß kann die Behandlung des Gasgemisches auch einen Reinigungsschritt zur Abtrennung von NOₓ aus dem Gasgemisch umfassen. Derartige Verfahren zur Abtrennung von NOₓ sind grundsätzlich aus dem Stand der Technik bekannt. Erfindungsgemäß können alle dem Fachmann bekannten Verfahren zur Abtrennung von NOₓ eingesetzt werden.

Erfindungsgemäß ist es dabei bevorzugt, dass das Abgas einer Behandlung umfassend eine Absorption in einem geeigneten Lösungsmittel und anschließende Desorption unterworfen wird, um inerte Verbindungen zu entfernen. Ein geeignetes Lösungsmittel ist beispielsweise Wasser, wie in der DT 20 40 219 beschrieben.

Gemäß einer beispielsweise bevorzugten Ausführungsform des erfindungsgemäßen Verfahrens ist es zur Aufkonzentrierung möglich, das oben genannte, Distickstoffmonoxid enthaltende Abgas mindestens einer Adsorptionskolonne zuzuführen und das Distickstoffmonoxid in mindestens einem organischen Lösungsmittel einzulösen. Als Lösungsmittel ist hierfür beispielsweise Cyclododecen geeignet. Diese erfindungsgemäße Verfahrensvariante bietet den Vorteil, dass die resultierende Lösung von Distickstoffmonoxid in Cyclododecen ohne weitere Aufarbeitung der erfindungsgemäßen Umsetzung zugeführt werden kann. Diese Lösung von Distickstoffmonoxid in Cyclododecen kann Distickstoffmonoxid in sämtlichen denkbaren Konzentrationen bis hin zur Sättigung enthalten. Gemäß anderer Ausführungsformen kann mindestens ein weiteres Lösungsmittel oder ein Gemisch aus Cyclododecen und mindestens einem weiteren Lösungsmittel zur Adsorption verwendet werden. Solche weiteren Lösungsmittel sind beispielsweise alle geeigneten gängigen organischen Lösungsmittel. Bevorzugte Lösungsmittel sind unter anderem N-Methylpyrrolidon, Dimethylformamid, Dimethylsulfoxid, Propylencarbonat, Sulfolan oder N,N-Dimethylacetamid. Wird mindestens ein weiteres Lösungsmittel oder ein Gemisch aus Cyclododecen und mindestens einem weiteren Lösungsmittel eingesetzt, so wird gemäß einer weiter bevorzugten Ausführungsform aus der mit Distickstoffmonoxid angereicherten Lösung in mindestens einem geeigneten Desorptionsschritt das Distickstoffmonoxid zumindest teilweise, bevorzugt im wesentlichen vollständig gewonnen und der erfindungsgemäßen Umsetzung zugeführt.

Gemäß einer weiteren Ausführungsform kann die chemische Zusammensetzung eines Abgases auch durch Zusatz von reinem Distickstoffmonoxid zu dem Abgas verändert werden.

Gemäß einer weiter bevorzugten Ausführungsform der vorliegenden Erfindung stammt das mindestens eine Distickstoffmonoxid enthaltende Abgas aus einer Adipinsäureanlage, einer Dodecandisäureanlage, einer Hydroxylaminanlage oder und/einer Salpetersäureanlage, wobei letztere wiederum bevorzugt mit mindestens einem Abgas einer Adipinsäureanlage, einer Dodecandisäureanlage oder einer Hydroxylaminanlage betrieben wird.

Gemäß einer bevorzugten Ausführungsform wird der Abgasstrom einer Adipinsäureanlage eingesetzt, bei der durch Oxidation von Cyclohexanol/Cyclohexanon-Gemischen mit Salpetersäure pro Mol gebildeter Adipinsäure im Allgemeinen 0,8 bis 1,0 mol N₂O gebildet werden. Wie beispielsweise in A. K. Uriarte et al., Stud. Surf. Sci. Catal. 130 (2000) S. 743-748 beschrieben, enthalten die Abgase von Adipinsäureanlagen in unterschiedlichen Konzentrationen noch weitere Bestandteile wie unter anderem Stickstoff, Sauerstoff, Kohlendioxid, Kohlenmonoxid, Stickoxide, Wasser und flüchtige organische Verbindungen.

Bei der obenstehend erwähnten Dodecandisäureanlage handelt es sich um den im Wesentlichen identischen Anlagentyp.

Eine beispielsweise typische Zusammensetzung eines Abgases einer Adipinsäureanlage oder einer Dodecandisäureanlage ist in der folgenden Tabelle wiedergegeben:

| Komponente | Konzentrationen / Gew.-% |
|---|---|
| NOₓ | 19 - 25 |
| N₂O | 20 - 28 |
| N₂ | 30 - 40 |
| O₂ | 7 - 10 |
| CO₂ | 2-3 |
| H₂O | ~7 |

Der Abgasstrom einer Adipinsäureanlage oder einer Dodecandisäureanlage kann direkt in dem erfindungsgemäßen Verfahren eingesetzt werden. Bevorzugt wird der Abgasstrom der Adipinsäureanlage oder einer Dodecandisäureanlage vor dem Einsatz zur Umsetzung des Cyclododecens gereinigt. Dabei wird beispielsweise vorteilhaft der Gehalt des Abgasstroms an Sauerstoff und/oder Stickoxiden auf Gehalte im Bereich von jeweils 0 bis 0,5 Vol.-% eingestellt. In der oben zitierten Schrift von A. K. Uirarte et al. werden verschiedene Möglichkeiten offenbart, wie ein solcher Abgasstrom zur Verwendung bei der katalytischen Benzolhydroxylierung gereinigt werden kann. Es werden Absorptionsverfahren wie beispielsweise Druckwechselabsorption, Membran-Trennverfahren, Tieftemperaturdestillation oder eine Kombination aus selektiver katalytischer Reduktion mit Ammoniak, gefolgt von der katalytischen Entfernung von Sauerstoff beschrieben. Sämtliche dieser Reinigungsmethoden sind auch anwendbar, um den Distickstoffmonoxid enthaltenden Abgasstrom einer industriellen Anlage wie beispielsweise einer Adipinsäureanlage oder einer Dodecandisäureanlage oder einer Salpetersäureanlage zu reinigen. Ganz besonders bevorzugt sind die destillative Reinigung und damit die destillative Aufkonzentrierung des Abgasstroms einer Adipinsäureanlage oder einer Dodecandisäureanlage oder einer Salpetersäureanlage.

Besonders bevorzugt wird im Rahmen der vorliegenden Erfindung der Abgasstrom einer Adipinsäureanlage oder einer Dodecandisäureanlage im Falle, dass dieser Sauerstoff und/oder Stickoxide zu jeweils mehr als 0,5 Vol.-% enthält, aufgereinigt.

Demgemäß beschreibt die vorliegende Erfindung auch ein wie oben beschriebenes Verfahren, das dadurch gekennzeichnet ist, dass zur Umsetzung des Cyclododecens der Abgasstrom einer Adipinsäureanlage oder einer Dodecandisäureanlage eingesetzt wird.

Weiter beschreibt die vorliegende Erfindung demgemäß ein wie oben beschriebenes Verfahren, das dadurch gekennzeichnet ist, dass der gegebenenfalls bevorzugt destillativ gereinigte Abgasstrom der Adipinsäureanlage oder einer Dodecandisäureanlage Sauerstoff und/oder Stickoxide im Bereich von jeweils 0 bis 0,5 Vol.-% enthält.

Gemäß einer ebenfalls bevorzugten Ausführungsform wird der Abgasstrom einer Salpetersäureanlage eingesetzt, die ganz oder teilweise mit Distickstoffmonoxid und Stickoxide enthaltenden Abgasen aus anderen Verfahren gespeist wird. In derartigen Salpetersäureanlagen werden Stickoxide adsorbiert und zum größten Teil zu Salpetersäure umgesetzt, während Distickstoffmonoxid nicht umgesetzt wird. Beispielsweise kann eine derartige Salpetersäureanlage durch Stickoxide, die durch gezielte Verbrennung von Ammoniak hergestellt werden, und durch Abgase einer Adipinsäureanlage und/oder durch Abgase einer Dodecandisäureanlage gespeist werden. Ebenso ist es möglich, eine derartige Salpetersäureanlage allein durch Abgase einer Adipinsäureanlage und/oder durch Abgase einer Dodecandisäureanlage zu speisen.

Die Abgase von derartigen Salpetersäureanlagen enthalten grundsätzlich in unterschiedlichen Konzentrationen noch weitere Bestandteile wie unter anderem Stickstoff, Sauerstoff, Kohlendioxid, Kohlenmonoxid, Stickoxide, Wasser und flüchtige organische Verbindungen.

Eine beispielsweise typische Zusammensetzung eines Abgases einer derartigen Salpetersäureanlage ist in der folgenden Tabelle wiedergegeben:

| Komponente | Konzentrationen / Gew.-% |
|---|---|
| NOₓ | < 0,1 |
| N₂O | 8 - 36 |
| N₂ | 57 - 86 |
| O₂ | 3-9 |
| CO₂ | 1-4 |
| H₂O | ~0,6 |

Der Abgasstrom einer Salpetersäureanlage kann direkt in dem erfindungsgemäßen Verfahren eingesetzt werden. Bevorzugt wird der Abgasstrom der Salpetersäureanlage vor dem Einsatz zur Umsetzung des Cyclododecens gereinigt. Dabei wird beispielsweise vorteilhaft der Gehalt des Abgasstroms an Sauerstoff und/oder Stickoxiden auf Gehalte im Bereich von jeweils 0 bis 0,5 Vol.-% eingestellt. Geeignete Verfahren, mit denen diese Werte einstellbar sind, sind obenstehend im Rahmen der Adipinsäureanlage und Dodecandisäureanlage beschrieben. Ganz besonders bevorzugt sind auch im Rahmen der Abgase der Salpetersäureanlage die destillative Reinigung und damit die destillative Aufkonzentrierung.

Besonders bevorzugt wird im Rahmen der vorliegenden Erfindung der Abgasstrom einer Salpetersäureanlage im Falle, dass dieser Sauerstoff und/oder Stickoxide zu jeweils mehr als 0,5 Vol.-% enthält, aufgereinigt.

Demgemäß beschreibt die vorliegende Erfindung auch ein wie oben beschriebenes Verfahren, das dadurch gekennzeichnet ist, dass zur Umsetzung des Cyclododecens der Distickstoffmonoxid enthaltende Abgasstrom einer Salpetersäureanlage eingesetzt wird.

Weiter beschreibt die vorliegende Erfindung demgemäß ein wie oben beschriebenes Verfahren, das dadurch gekennzeichnet ist, dass der gegebenenfalls bevorzugt destillativ gereinigte Abgasstrom der Salpetersäureanlage Sauerstoff und/oder Stickoxide in einem Bereich von 0 bis 0,5 Vol.-% enthält.

Gemäß einer ebenfalls bevorzugten Ausführungsform des erfindungsgemäßen Verfahrens wird der Abgasstrom einer Hydroxylaminanlage eingesetzt, bei der beispielsweise zunächst Ammoniak mit Luft oder Sauerstoff zu NO oxidiert wird, wobei kleine Mengen an Distickstoffmonoxid als Nebenprodukt gebildet werden. Das NO wird anschließend mit Wasserstoff zu Hydroxylamin hydriert. Nachdem Distickstoffmonoxid unter den Hydrierbedingungen inert ist, reichert es sich im Wasserstoffkreis an. In bevorzugten Verfahrensführungen enthält der Purge-Strom einer Hydroxylaminanlage Distickstoffmonoxid im Bereich von 9 bis 13 Vol.-% in Wasserstoff. Dieser Purge-Strom kann als solcher zur erfindungsgemäßen Umsetzung eingesetzt werden. Ebenso ist es möglich, diesen Strom bezüglich des Distickstoffmonoxidgehaltes, wie oben beschrieben, geeignet aufzukonzentrieren.

Demgemäß betrifft die vorliegende Erfindung auch ein wie oben beschriebenes Verfahren, das dadurch gekennzeichnet ist, dass die Distickstoffmonoxidquelle das Abgas einer Adipinsäureanlage und/oder einer Dodecandisäureanlage und/oder einer Hydroxylaminanlage und/oder einer mit dem Abgas einer Adipinsäureanlage und/oder einer Dodecandisäureanlage und/oder einer Hydroxylaminanlage betriebenen Salpetersäureanlage ist.

Ebenso beschreibt die vorliegende Erfindung auch in integriertes Verfahren zur Herstellung von Cyclododecanon, das mindestens die folgenden Schritte (i) und (ii) umfasst:
(i) Bereitstellung eines Distickstoffmonoxid enthaltenden Gasgemisches, enthaltend jeweils von 0 bis 0,5 Vol.-% Sauerstoff und/oder Stickoxide, auf der Basis mindestens eines Abgasstroms mindestens einer Adipinsäureanlage und/oder mindestens einer Dodecandisäureanlage und/oder mindestens einer Hydroxylaminanlage und/oder mindestens einer mit dem Abgas einer Adipinsäureanlage und/oder einer Dodecandisäureanlage und/oder einer Hydroxylaminanlage betriebenen Salpetersäureanlage;
(ii) Umsetzung von Cyclododecen mit dem gemäß (i) bereitgestellten Gasgemisch unter Erhalt von Cyclododecanon.

Ebenso ist es möglich, Distickstoffmonoxid gezielt herzustellen. Möglich ist unter anderem die Herstellung über die thermische Zersetzung von NH₄NO₃, wie dies beispielsweise in der US 3,656,899 beschrieben ist. Ebenfalls möglich ist die Herstellung über die katalytische Oxidation von Ammoniak, wie dies beispielsweise in der US 5,849,257 oder in der WO 98/25698 beschrieben ist.

Im Rahmen der erfindungsgemäßen Umsetzung des Cyclododecens mit Distickstoffmonoxid kann mindestens ein geeignetes Lösungsmittel oder Verdünnungsmittel eingesetzt werden. Als solche sind unter anderem Cyclododecan oder Cyclododecanon zu nennen, wobei im Wesentlichen alle gängigen Lösungs- und/oder Verdünnungsmittel geeignet sind unter der Maßgabe, dass sie weder eine C-C-Doppelbindung noch eine C-C-Dreifachbindung noch eine Aldehydgruppe aufweisen.

Im Allgemeinen ist bei der erfindungsgemäßen Umsetzung mit Distickstoffmonoxid der Zusatz eines Lösungsmittels oder Verdünnungsmittel nicht notwendig.

Die Umsetzung des Cyclododecens mit Distickstoffmonoxid kann kontinuierlich oder in Batch-Fahrweise durchgeführt werden, wobei auch Kombinationen aus kontinuierlicher und Batch-Fahrweise möglich sind. Bevorzugt ist die kontinuierliche Verfahrensführung.

Als Reaktoren sind sämtliche geeigneten Reaktoren zu nennen. Beispielsweise kann die Umsetzung von Cyclododecen mit Distickstoffmonoxid oder einem Distickstoffmonoxid enthaltenden Gasgemisch in mindestens einem CSTR (Continuous Stirred Tank Reactor) mit internem oder externem Wärmetauscher, in mindestens einem Rohrreaktor, in mindestens einem Schlaufenreaktor oder einer Kombination aus mindestens zwei dieser Reaktoren durchgeführt werden. Ebenso ist es möglich, mindestens einen dieser Reaktoren so auszugestalten, dass er mindestens zwei unterschiedliche Zonen aufweist. Solche Zonen können sich beispielsweise in Reaktionsbedingungen wie beispielsweise der Temperatur oder dem Druck und/oder in der Geometrie der Zone wie beispielsweise dem Volumen oder dem Querschnitt unterscheiden. Denkbar ist auch ein axiales Temperaturprofil, das beispielsweise durch Gleichstromkühlung und entsprechende Einstellung der Kühlmittelmenge zu realisieren ist.

Besonders bevorzugt wird die Oxidation des Cyclododecens in mindestens einem Rohrreaktor durchgeführt.

Demgemäß beschreibt die vorliegende Erfindung auch ein wie oben beschriebenes Verfahren, das dadurch gekennzeichnet ist, dass die Umsetzung des Cyclododecens mit Distickstoffmonoxid in mindestens einem Rohrreaktor durchgeführt wird.

Die Umsetzung des Cyclododecens erfolgt bevorzugt bei einer Temperatur im Bereich von 140 bis 350 °C, weiter bevorzugt im Bereich von 200 bis 325 °C und besonders bevorzugt im Bereich von 225 bis 300 °C.

Demgemäß betrifft die vorliegende Erfindung auch ein wie oben beschriebenes Verfahren, das dadurch gekennzeichnet ist, dass die Umsetzung kontinuierlich in mindestens einem Rohrreaktor bei einer Temperatur im Bereich von 140 bis 350 °C durchgeführt wird.

Der Druck im Reaktionsgefäß, bevorzugt in mindestens einem Rohrreaktor, liegt im Allgemeinen bei Werten, die größer oder gleich, bevorzugt größer dem Eigendruck des Eduktgemisches beziehungsweise des Produktgemisches bei der gewählten Reaktionstemperatur oder den gewählten Reaktionstemperaturen im Reaktionsgefäß sind. Im Allgemeinen liegen die Reaktionsdrücke im Bereich von 1 bis 14.000 bar, bevorzugt im Bereich vom Eigendruck bis 3000 bar, besonders bevorzugt im Bereich vom Eigendruck bis 1000 bar und insbesondere bevorzugt im Bereich vom Eigendruck bis 500 bar.

Die Verweilzeit des Reaktionsgutes im Reaktor liegt im Allgemeinen bei bis zu 30 h, bevorzugt im Bereich von 0,1 bis 30 h, weiter bevorzugt im Bereich von 0,25 bis 25 h, besonders bevorzugt im Bereich von 0,3 bis 20 h und insbesondere bevorzugt im Bereich von 0,5 bis 20 h.

Das Molverhältnis der Edukte Distickstoffmonoxid : Cyclododecen liegt im Allgemeinen bei bis zu 5:1, bevorzugt im Bereich von 1:1 bis 4:1, weiter bevorzugt im Bereich von 1:1 bis 3:1 und besonders bevorzugt im Bereich von 1,01:1 bis 2:1. Gemäß einer alternativen Ausführungsform liegt das Molverhältnis beispielsweise bei 0,5 bis 5, insbesondere bei 0,07 bis 2, bevorzugt bei 0,1 bis 2, besonders bevorzugt bei 0,1 bis 1.

Insbesondere bevorzugt werden die Reaktionsbedingungen so gewählt, dass der Umsatz an Cyclododecen im Bereich von 30 bis 95 %, besonders bevorzugt im Bereich von 40 bis 85 % und insbesondere bevorzugt im Bereich von 50 bis 80 % liegt. Gemäß einer alternativen Ausführungsform werden die Reaktionsbedingungen so gewählt, dass der Umsatz an Cyclododecen im Bereich von 5 bis 95 % liegt, bevorzugt bei 7 bis 80 %, insbesondere bei 10 bis 50 %.

Der Begriff "Umsatz", wie er obenstehend verwendet wird, bezeichnet den Gesamtumsatz an Cyclododecen. Wird als Edukt ausschließlich cis-Cyclododecen oder ausschließlich trans-Cyclododecen eingesetzt, so entspricht der Gesamtumsatz dem Umsatz an dem jeweiligen Isomer. Wird als Edukt ein Gemisch aus cis- und trans-Isomer, enthaltend x Mol.-% cis-Isomer und y Mol.-% trans-Isomer, eingesetzt und das cis-Isomer zu m % und das trans-Isomer zu n % umgesetzt, so berechnet sich der Gesamtumsatz als Summe mx + ny.

Im Falle, dass als Edukt ein Isomerengemisch eingesetzt wird, ist es im Rahmen der vorliegenden Erfindung bevorzugt, die Umsetzung mit Distickstoffmonoxid in mindestens zwei Schritten, weiter bevorzugt in zwei oder drei Schritten und ganz besonders bevorzugt in zwei Schritten durchzuführen.

In einem ersten Schritt wird dabei eine Temperatur gewählt, die bevorzugt im Bereich von 140 bis 300 °C, weiter bevorzugt im Bereich von 180 bis 290 °C und insbesondere bevorzugt im Bereich von 225 bis 275 °C liegt. In diesem ersten Schritt wird hauptsächlich das trans-Isomer zu Cyclododecanon oxidiert. In einem zweiten Schritt wird eine im Vergleich zum ersten Schritt erhöhte Temperatur gewählt, die bevorzugt im Bereich von 165 bis 350 °C, weiter bevorzugt im Bereich von 225 bis 325 °C und insbesondere bevorzugt im Bereich von 275 bis 310 °C liegt. In diesem zweiten Schritt wird das cis-Isomer zu Cyclododecanon oxidiert.

Demgemäß betrifft die vorliegende Erfindung auch ein wie oben beschriebenes Verfahren, das dadurch gekennzeichnet ist, dass ein Gemisch, enthaltend cis-Cyclododecen und trans-Cyclododecen, mit Distickstoffmonoxid in zwei Stufen umgesetzt wird.

Ebenso betrifft die vorliegende Erfindung auch ein wie oben beschriebenes Verfahren, das dadurch gekennzeichnet ist, dass in der ersten Stufe die Umsetzung bei einer Temperatur im Bereich von 140 bis 300 °C und in der zweiten Stufe die Umsetzung bei einer Temperatur im Bereich von 165 bis 350 °C durchgeführt wird, wobei die Temperatur in der ersten Stufe niedriger ist als die Temperatur in der zweiten Stufe.

Was die weiteren Reaktionsparameter wie beispielsweise Druck, Verweilzeit oder Reaktionsgefäße der beiden Stufen des oben genannten bevorzugten zweistufigen Verfahrens anbelangt, so wird diesbezüglich auf die allgemeinen und bevorzugten Ausführungsformen des oben beschriebenen einstufigen Verfahrens verwiesen.

Das oben beschriebene zweistufige Verfahren kann gemäß sämtlicher geeigneter Verfahrensführungen realisiert werden. Beispielsweise kann das zweistufige Verfahren in mindestens zwei Reaktoren durchgeführt werden, wobei in mindestens einem Reaktor die niedrigere Temperatur und in mindestens einem weiteren Reaktor die höhere Temperatur eingestellt wird. Ebenso ist es möglich, die unterschiedlichen Temperaturen in einem einzigen Reaktor, der mindestens zwei Zonen verschiedener Temperatur aufweist, zu realisieren. Wird ein Reaktor mit mindestens zwei Zonen verschiedener Temperatur eingesetzt, so können die beiden Temperaturen kontinuierlich oder diskontinuierlich ineinander übergehen. Beispielsweise besonderes bevorzugt ist hierbei ein Rohrreaktor mit einem axialen Temperaturprofil, das beispielsweise wie oben beschrieben zu realisieren ist.

Werden im Rahmen des zweistufigen Verfahrens mindestens zwei verschiedene Reaktoren eingesetzt, so kann zwischen mindestens zwei dieser Reaktoren mindestens eine Zwischenbehandlung des Reaktionsgutes erfolgen. Als mögliche Zwischenbehandlungen sind etwa zu nennen:
- Erwärmung des Reaktionsgutes;
- Änderung des Druckes, unter dem sich das Reaktionsgut befindet. Bevorzugt ist in diesem Zusammenhang beispielsweise die Erhöhung des Druckes über beispielsweise mindestens eine Pumpe und/oder mindestens einen Kompressor;
- Zudosierung mindestens eines Edukts. Insbesondere kann Distickstoffmonoxid und/oder Cyclododecen zudosiert werden. Im Falle des Cyclododecens kann es sich dabei um frisches Edukt und/oder um Cyclododecen handeln, das in der zweiten Stufe nicht umgesetzt wird und durch mindestens eine geeignete Maßnahme aus dem Produktstrom abgetrennt und in das Verfahren rückgeführt wird.
- Abtrennung von gebildetem Cyclododecanon durch mindestens eine geeignete Maßnahme wie beispielsweise bevorzugt durch mindestens einen destillativen Schritt.

Gemäß einer weiter bevorzugten Ausführungsform des erfindungsgemäßen Verfahrens wird im Falle, dass als Edukt ein Gemisch aus cis- und trans-Cyclododecen eingesetzt wird, mindestens ein Katalysator zugegeben, der in der Lage ist, unter den Reaktionsbedingungen, die für die Umsetzung des Cyclododecens gewählt werden, die Einstellung des Gleichgewichts zwischen cis- und trans-Isomer zu katalysieren.

Grundsätzlich können hierfür sämtliche geeigneten Katalysatoren eingesetzt werden. Besonders bevorzugt wird im erfindungsgemäßen Verfahren zu diesem Zweck mindestens ein Katalysator eingesetzt, wie er auch für Hydrierungen beispielsweise von Olefinen oder Polyenen verwendet wird. Insbesondere bevorzugt werden im erfindungsgemäßen Verfahren solche Isomerisierungskatalysatoren eingesetzt, die mindestens ein Übergangsmetall wie unter anderem bevorzugt Ru enthalten.

Die zur Gleichgewichtseinstellung zwischen cis- und trans-Isomer eingesetzten lsomerisierungskatalysatoren können entweder homogene oder heterogene Katalysatoren sein. Ebenso ist es möglich, mindestens einen homogenen und mindestens einen heterogenen Katalysator einzusetzen. Die heterogenen Katalysatoren können hierbei als Suspensions- oder als Festbettkatalysator eingesetzt werden. Ebenso ist es möglich, sowohl mindestens einen heterogenen Suspensionskatalysator als auch mindestens einen heterogenen Festbettkatalysator, gegebenenfalls zusätzlich zu mindestens einem homogenen Katalysator, einzusetzen. Besonders bevorzugt wird mindestens ein homogener Katalysator eingesetzt.

Während grundsätzlich sämtliche geeigneten homogenen Katalysatoren eingesetzt werden können, werden bevorzugt solche verwendet, die Ru als aktives Metall enthalten. Weiter besonders bevorzugt werden Katalysatoren eingesetzt, wie sie in der US 5,180,870, US 5,321,176, US 5,177,278, US 3,804,914, US 5,210,349 US 5,128,296, US B 316,917 und in D.R. Fahey in J. Org. Chem. 38 (1973) S. 80-87 beschrieben sind. Solche Katalysatoren sind etwa (TPP)₂(CO)₃Ru, [Ru(CO)₄]₃, (TPP)₂Ru(CO)₂Cl₂, (TPP)₃(CO)RuH₂, (TPP)₂(CO)₂RuH₂, (TPP)₂(CO)₂RuClH, oder (TPP)₃(CO)RuCl₂.

Als ganz besonders bevorzugter Katalysator wird (TPP)₂(CO)₂RuCl₂ oder eine entsprechende CI-freie Variante wie beispielsweise (TPP)₂(CO)₂RuH₂ eingesetzt, wobei TPP für Triphenylphosphin steht.

Gemäß einer weiter bevorzugten Ausführungsform wird der eingesetzte Katalysator im erfindungsgemäßen Verfahren in situ hergestellt. Bei dieser Herstellung in situ geht man beispielsweise bevorzugt von den Verbindungen Rutheniumchlorid, Rutheniumacetat, Rutheniumacetylacetonat oder anderen Ru-Verbindungen aus.

Im Allgemeinen wird der Oxidation außer der mindestens einen Ru-Komponente zusätzlich mindestens eine der Verbindungen NR₃, PR₃, AsR₃ oder SbR₃ zugesetzt, wobei R für einen Alkyl-, Aralkyl-, Alkaryl- oder Arylrest mit bevorzugt 1 bis 20 C-Atomen steht. Insbesondere bevorzugt ist im Rahmen der vorliegenden Erfindung hierbei Triphenylphosphin.

Im Rahmen einer weiteren Ausführungsform wird die Oxidation in Gegenwart mindestens einer Carbonsäure durchgeführt, wie dies in der DE 198 56 862 A1 beschrieben ist.

Als Carbonsäure können beispielsweise aliphatische, cycloaliphatische, aromatische oder araliphatische Carbonsäuren verwendet werden. Bevorzugt werden solche eingesetzt, die unter den Reaktionsbedingungen im Reaktionssystem löslich sind. Beispiele sind etwa C₁-C₂₀ Monocarbonsäuren, C₂-C₆ Dicarbonsäuren, Cyclohexylcarbonsäure, Benzoesäure, Terephthalsäure, Phthalsäure oder Phenylessigsäure. Besonders bevorzugte Säuren sind aliphatische Mono- und Dicarbonsäuren, insbesondere Essigsäure, Propionsäure sowie C₁₂C₂₀-Fettsäuren, Bernsteinsäure und Adipinsäure.

Bei der in situ-Herstellung des Katalysators wird besonders bevorzugt noch mindestens eine CO-Quelle zugesetzt. Diese kann CO selbst sein. Weitere mögliche CO-Quellen sind beispielsweise Formaldehyd, Methanol, Ethanol oder andere geeignete primäre Alkohole wie beispielsweise Benzylalkohol oder Diole oder Polyole mit mindestens einer primären Alkoholgruppe wie beispielsweise Ethylenglykol, Propylenglykol oder Glycerin.

Aus der erfindungsgemäßen Oxidation des Cyclododecens resultiert im Allgemeinen ein Produktgemisch. Bevorzugt enthält dieses Produktgemisch im Bereich von 30 bis 95 Gew.-%, besonders bevorzugt von 40 bis 90 Gew.-% und insbesondere bevorzugt von 50 bis 80 Gew.-% Cyclododecanon, jeweils bezogen auf das Gesamtgewicht des Produktgemisches nach Abkühlung auf 20 °C und Entspannung auf Normaldruck. Gemäß einer alternativen Ausführungsform enthält das Produktgemisch beispielsweise von 5 bis 95 Gew.-% Cyclododecanon, bevorzugt von 7 bis 80 Gew.-%, insbesondere von 10 bis 50 Gew.-%.

Als weitere Bestandteile enthält das Produktgemisch gegebenenfalls vor der Oxidationsstufe verwendeten und nicht abgetrennten Katalysator, nicht umgesetztes Cyclododecen sowie gegebenenfalls mit dem Edukt in die Oxidation eingebrachte Verbindungen, wie beispielsweise Cyclododecan, und bei der Umsetzung mit Distickstoffmonoxid gegebenenfalls mit umgesetzte Verbindungen wie dies untenstehend beschrieben ist.

Der zur Umsetzung verwendete lsomerisierungskatalysator kann im Folgenden entweder in das Verfahren rückgeführt werden, verworfen werden oder aufgearbeitet werden, beispielsweise, um das im Katalysator enthaltene mindestens eine Metall zurückzugewinnen. Wird der Katalysator in das Verfahren rückgeführt, so kann dieser entweder in die Verfahrensstufe der Umsetzung mit Distickstoffmonoxid zurückgeführt werden oder in einen beliebigen anderen Schritt, den das erfindungsgemäße Verfahren zusätzlich aufweisen kann. Gemäß einer untenstehend beschriebenen besonders bevorzugten Ausführungsform weist das erfindungsgemäße Verfahren als solchen zusätzlichen Schritt die Partialhydrierung mindestens eines Cyclododecatriens auf, wobei die genannte Partialhydrierung weiter bevorzugt in Anwesenheit des gleichen Katalysators erfolgen kann, der als Isomerisierungskatalysator zur Gleichgewichtseinstellung zwischen cis- und trans-Isomer des Cyclododecens eingesetzt wird. Auch dieser Partialhydrierung kann demgemäß der abgetrennte Katalysator zugeführt werden, wobei der Katalysator vor der Zuführung einem geeigneten Regenerationsschritt unterworfen werden kann.

Das als Edukt eingesetzte Cyclododecen, das entweder als cis-Isomer oder als trans-Isomer oder als Gemisch aus cis- und trans-Isomer eingesetzt werden kann, kann grundsätzlich aus jeder beliebigen Quelle stammen.

Ganz besonders bevorzugt wird im Rahmen der vorliegenden Erfindung Cyclododecen durch Partialhydrierung mindestens eines Cyclododecatriens, bevorzugt durch Partialhydrierung mindestens eines 1,5,9-Cyclododecatriens wie beispielsweise cis,trans,trans-1,5,9-Cyclododecatrien oder cis,cis,trans-1,5,9-Cyclododecatrien oder all-trans-1,5,9-Cyclododecatrien und insbesondere aus cis,trans,trans-1,5,9-Cyclododecatrien hergestellt.

Diese bevorzugten Verbindungen können beispielsweise durch Trimerisierung von reinem 1,3-Butadien hergestellt werden, wie dies beispielsweise in T. Schiffer, G. Oenbrink, "Cyclodecatriene, Cyclooctadiene, and 4-Vinylcyclohexene", Ullmann's Encyclopedia of Industrial Chemistry, 6th Edition (2000), Electronic Release, Wiley VCH beschrieben ist. Im Rahmen dieses Verfahrens entstehen beispielsweise bei Trimerisierung in Anwesenheit von Ziegler-Katalysatoren cis,trans,trans-1,5,9-Cyclododecatrien, cis,cis,trans-1,5,9-Cyclododecatrien und all-trans-1,5,9-Cyclododecatrien, wie dies beispielsweise in H. Weber et al. "Zur Bildungsweise von cis,trans,trans-Cyclododecatrien-(1.5.9) mittels titanhaltiger Katalysatoren" in: Liebigs Ann. Chem. 681 (1965) S.10-20 beschrieben ist. Während sämtliche dieser Cyclododecatriene im Rahmen des erfindungsgemäßen Verfahrens partialhydriert werden können, ist, wie oben beschrieben, die Umsetzung von cis,trans,trans-1,5,9-Cyclododecatrien im Rahmen des vorliegenden erfindungsgemäßen Verfahrens besonders bevorzugt. Dieses cis,trans,trans-1,5,9-Cyclododecatrien wird besonders bevorzugt gemäß des in dem oben erwähnten Artikel von Weber et al. hergestellt.

Demgemäß beschreibt die vorliegende Erfindung auch ein wie oben beschriebenes Verfahren, das dadurch gekennzeichnet ist, dass das Cyclododecatrien durch Trimerisierung von 1,3-Butadien unter Verwendung eines Titan-Katalysators hergestellt wird.

Während grundsätzlich sämtliche geeigneten Titan-Katalysatoren zur Trimerisierung eingesetzt werden können, ist der im Artikel von Weber et al. beschriebene Titantetrachlorid/Ethylaluminiumsesquichlorid-Katalysator besonders bevorzugt.

Das für die Trimerisierung eingesetzte Butadien weist insbesondere bevorzugt einen über Gaschromatographie bestimmten Reinheitsgrad von mindestens 99,6 % und weiter bevorzugt von mindestens 99,65 % auf. Insbesondere bevorzugt enthält das eingesetzte 1,3-Butadien im Rahmen der Nachweisgenauigkeit kein 1,2-Butadien und kein 2-Butin.

Aus dieser bevorzugten Trimerisierung werden im Allgemeinen Gemische erhalten, die mindestens 95 Gew.-%, bevorzugt mindestens 96 Gew.-% und weitere bevorzugt mindestens 97 Gew.-% cis,trans,trans-1,5,9-Cyclododecatrien enthalten. Beispielsweise insbesondere bevorzugt enthalten die Gemische in etwa 98 Gew.-% cis,trans,trans-1,5,9-Cyclododecatrien.

Daher betrifft die vorliegende Erfindung auch ein Verfahren zur Herstellung von Cyclododecanon, das die Schritte (1) und (II) umfasst:
(I) Herstellung von Cyclododecen durch Partialhydrierung von Cyclododecatrien;
(II) Umsetzung von gemäß (I) erhaltenem Cyclododecen mit Distickstoffmonoxid unter Erhalt von Cyclododecanon,
wobei als Quelle des gemäß (II) eingesetzten Distickstoffmonoxids mindestens ein Distickstoffmonoxid enthaltendes Abgas mindestens eines industriellen Verfahrens dient. Ebenso betrifft die vorliegende Erfindung auch ein Verfahren zur Herstellung von Cyclododecanon, das die Schritte (I) und (II) umfasst und das dadurch gekennzeichnet ist, dass das in Schritt (I) eingesetzte Cyclododecatrien durch Trimerisierung von 1,3-Butadien hergestellt wird.

Insbesondere betrifft die vorliegende Erfindung auch ein Verfahren zur Herstellung von Cyclododecanon, das die Schritte (I) und (II) umfasst, wobei das in Schritt (I) eingesetzte Cyclododecatrien durch Trimerisierung von 1,3-Butadien hergestellt wird, das dadurch gekennzeichnet ist, dass die Trimerisierung in Anwesenheit eines Titan-Katalysators erfolgt und das Cyclododecatrien cis,trans,trans-1,5,9-Cyclododecatrien ist.

Ebenso betrifft die vorliegende Erfindung auch ein wie oben beschriebenes Verfahren, das dadurch gekennzeichnet ist, dass das Cyclododecen aus der katalytischen Partialhydrierung von Cyclododecatrien erhalten wird.

Weiter beschreibt die vorliegende Erfindung auch ein integriertes Verfahren zur Herstellung von Cyclododecanon, das mindestens die folgenden Schritte (a) und (b) sowie (i) und (ii) umfasst:
(a) Herstellung von Cyclododecatrien aus 1,3-Butadien;
(b) Partialhydrierung des Cyclododecatriens unter Erhalt von Cyclododecen;
(i) Bereitstellung eines Distickstoffmonoxid enthaltenden Gasgemisches, enthaltend jeweils von 0 bis 0,5 Vol.-% Sauerstoff und/oder Stickoxide, auf der Basis mindestens eines Abgasstroms einer Adipinsäureanlage und/oder einer Salpetersäureanlage und/oder einer Hydroxylaminanlage und/oder einer mit dem Abgas einer Adipinsäureanlage und/oder einer Dodecandisäureanlage und/oder einer Hydroxylaminanlage betriebenen Salpetersäureanlage;
(ii) Umsetzung von gemäß (b) erhaltenem Cyclododecen mit dem gemäß (i) bereitgestellten Gasgemisch unter Erhalt von Cyclododecanon.

Verfahren zur katalytischen Partialhydrierung von Cyclododecatrien sind in der Literatur beschrieben. Dabei ist es im Allgemeinen essentiell, dass die Ausbeute bei dieser Reaktion sehr hoch ist, da sich aufgrund der geringen Massen- und Polaritätsunterschiede der Edukte und Produkte diese nicht oder nur sehr aufwändig destillativ voneinander trennen lassen. Der Cyclododecatrien-Umsatz muss daher möglichst quantitativ sein.

Die Hydrierung von Polyenen zu Monoenen an homogenen Ru-Katalysatoren unter Zusatz von Wasser ist beispielsweise in der US 5,180,870 beschrieben. In Beispiel 2 dieser Schrift wird unter Zusatz von Wasser nach 4 h Reaktionszeit ein Cyclododecatrien-Umsatz von 98,4 % erreicht. Welche Cyclododecen-Ausbeute erhalten wird, ist nicht beschrieben. In Beispiel 1 dieser Schrift wird unter Zusatz von etwas weniger Wasser als im Beispiel 2 nach 8 h Reaktionszeit ein nur unbefriedigender Umsatz von 85,8 % erreicht.

In der US 5,321,176 ist der Zusatz von Aminen zur homogen katalysierten Hydrierung beschrieben.

In der US 5,177,278 wird die Cyclododecatrien-Hydrierung mit homogenen Ru-Katalysatoren in Gegenwart von Lösungsmitteln wie Ethern oder Estern vorgenommen. Nach den Beispielen dieser Schrift liegen die besten Cyclododecen-Selektivitäten bei 96 - 98 %. Allerdings wird in keinem Fall quantitativer Umsatz erreicht, so dass sich bei der Aufarbeitung ein Trennproblem stellt.

In der US 3,925,494 wird ebenfalls in Lösungsmitteln gearbeitet. Die maximale Cyclododecen-Ausbeute ist mit ca. 95 % beschrieben. Allerdings ist auch hier der Umsatz nicht quantitativ.

Von D. R. Fahey wird in J. Org. Chem. 38 (1973) S. 80-87 die Hydrierung von Cyclododecatrien an verschiedenen homogenen Ru-Katalysatoren beschrieben. In allen Beispielen wird in Gegenwart größerer Mengen Lösungsmittel gearbeitet. Es werden Cyclododecen-Ausbeuten von ca. 98 % beschrieben. Allerdings ist die beschriebene Einsatzmenge an Ru, bezogen auf Cyclododecatrien, sehr hoch.

In der DE 198 56 862 A1 ist die Hydrierung von Cyclododecatrien an homogenen Ru-Katalysatoren in Gegenwart von Carbonsäuren beschrieben. Dabei können Cyclododecen-Ausbeuten von 98 % erreicht werden.

Im Rahmen der vorliegenden Erfindung kann die katalytische Partialhydrierung von Cyclododecatrien zu Cyclododecen gemäß sämtlicher geeigneter Methoden erfolgen.

Insbesondere kann die katalytische Partialhydrierung mit homogenen oder heterogenen Katalysatoren durchgeführt werden, wobei die heterogenen Katalysatoren als Suspension oder als Festbett eingesetzt werden können.

Als heterogene Katalysatorsysteme werden bevorzugt solche eingesetzt, die mindestens eines der Elemente Pd, Pt, Ru, Ir, Ni und Rh als aktives Hydriermetall enthalten.

Gemäß einer besonders bevorzugten Ausführungsform wird im erfindungsgemäßen Verfahren Cyclododecatrien in Anwesenheit mindestens eines homogenen Hydrierkatalysators zu Cyclododecen partiell hydriert.

Während grundsätzlich sämtliche geeigneten homogenen Katalysatoren eingesetzt werden können, werden bevorzugt solche verwendet, die Ru als aktives Hydriermetall enthalten. Weiter besonders bevorzugt werden Katalysatoren eingesetzt, wie sie in der US 5,180,870, US 5,321,176, US 5,177,278, US 3,804,914, US 5,210,349 US 5,128,296, US B 316,917 und in D.R. Fahey in J. Org. Chem. 38 (1973) S. 80-87 beschrieben sind. Solche Katalysatoren sind etwa (TPP)₂(CO)₃Ru, [Ru(CO)₄]₃, (TPP)₂Ru(CO)₂Cl₂, (TPP)₃(CO)RuH₂, (TPP)₂(CO)₂RuH₂, (TPP)₂(CO)₂RuCIH oder (TPP)₃(CO)RuCl₂.

Als ganz besonders bevorzugter Katalysator wird (TPP)₂(CO)₂RuCl₂ oder eine entsprechende CI-freie Variante wie beispielsweise (TPP)₂(CO)₂RuH₂ eingesetzt, wobei TPP für Triphenylphosphin steht.

Gemäß einer weiter bevorzugten Ausführungsform wird der zur Partialhydrierung eingesetzte Katalysator im erfindungsgemäßen Verfahren in situ hergestellt. Bei dieser Herstellung in situ geht man beispielsweise bevorzugt von den Verbindungen Rutheniumchlorid, Rutheniumacetat, Rutheniumacetylacetonat oder anderen Ru-Verbindungen aus.

Im Allgemeinen wird der Hydrier-Reaktion außer der mindestens einen Ru-Komponente zusätzlich mindestens eine der Verbindungen NR₃, PR₃, AsR₃ oder SbR₃ zugesetzt, wobei R für einen Alkyl-, Aralkyl-, Alkaryl- oder Arylrest mit bevorzugt 1 bis 20 C-Atomen steht. Insbesondere bevorzugt ist im Rahmen der vorliegenden Erfindung hierbei Triphenylphosphin.

Bezogen auf 1 kg Cyclododecatrien werden im erfindungsgemäßen Verfahren, gerechnet als Metall, im allgemeinen 0,1 bis 2000 mg aktives Hydriermetall, besonders bevorzugt Ru, eingesetzt. Bevorzugt werden 1 bis 1000 mg und besonders bevorzugt 10 bis 200 mg eingesetzt.

Gemäß einer Ausführungsform des erfindungsgemäßen Verfahrens wird der Katalysator nach erfolgter Partialhydrierung aus dem Reaktionsgut abgetrennt. Der abgetrennte Katalysator wird gemäß einer weiteren Ausführungsform der vorliegenden Erfindung einem beliebigen Verfahren zugeführt und ganz besonders bevorzugt in das erfindungsgemäße Verfahren rückgeführt. Die Abtrennung des Katalysators erfolgt erfindungsgemäß besonders bevorzugt in mindestens einer Destillation, wobei das Produkt der Partialhydrierung, Cyclododecen, über Kopf und der Katalysator, gegebenenfalls mit Anteilen an Cyclododecen, über Sumpf abgetrennt wird.

Aufgrund der wie obenstehend beschrieben sehr niedrigen Mengen an Katalysatormaterial und der damit sehr niedrigen Kosten für den Katalysator ist es im erfindungsgemäßen Verfahren im Allgemeinen nicht notwendig, den Katalysator nach der Partialhydrierung aus der Reaktionsmischung abzutrennen und in das Verfahren rückzuführen.

Im Rahmen einer weiteren Ausführungsform wird die Partialhydrierung in Gegenwart mindestens einer Carbonsäure durchgeführt, wie dies in der DE 198 56 862 A1 beschrieben ist.

Als Carbonsäure können beispielsweise aliphatische, cycloaliphatische, aromatische oder araliphatische Carbonsäuren verwendet werden. Bevorzugt werden solche eingesetzt, die unter den Reaktionsbedingungen im Reaktionssystem löslich sind. Beispiele sind etwa C₁-C₂₀ Monocarbonsäuren, C₂-C₆ Dicarbonsäuren, Cyclohexylcarbonsäure, Benzoesäure, Terephthalsäure, Phthalsäure oder Phenylessigsäure. Besonders bevorzugte Säuren sind aliphatische Mono- und Dicarbonsäuren, insbesondere Essigsäure, Propionsäure sowie C₁₂-C₂₀-Fettsäuren, Bernsteinsäure und Adipinsäure.

Die Menge an zugesetzter Säure pro kg Cyclododecatrien liegt im Allgemeinen bei 0,001 bis 100 g, bevorzugt bei 0,01 bis 50 g und besonders bevorzugt bei 0,05 bis 25 g.

Bei der in situ-Herstellung des Katalysators wird besonders bevorzugt noch mindestens eine CO-Quelle zugesetzt. Diese kann CO selbst sein. Weitere mögliche CO-Quellen sind beispielsweise Formaldehyd, Methanol, Ethanol oder andere geeignete primäre Alkohole wie beispielsweise Benzylalkohol oder Diole oder Polyole mit mindestens einer primären Alkoholgruppe wie beispielsweise Ethylenglykol, Propylenglykol oder Glycerin.

Die Partialhydrierung findet im erfindungsgemäßen Verfahren im Allgemeinen bei Temperaturen im Bereich von 50 bis 300 °C, bevorzugt im Bereich von 80 bis 250 °C und besonders bevorzugt im Bereich von 100 bis 200 °C statt. Die Reaktionsdrücke liegen dabei im Bereich von 1 bis 300 bar, bevorzugt im Bereich von 1 bis 200 bar und besonders bevorzugt im Bereich von 1 bis 100 bar.

Die Reaktionszeiten pro Ansatz in Batch-Fahrweise beziehungsweise die Verweilzeiten bei kontinuierlicher Verfahrensführung liegen im Allgemeinen im Bereich von 0,5 bis 48 h. Sie richten sich im Wesentlichen nach den Ansatzgrößen und den Möglichkeiten, Energie zuführen beziehungsweise abführen zu können. Durch den obenstehend beschriebenen Carbonsäurezusatz ist es nicht kritisch, wenn der Reaktionsansatz länger als erforderlich unter Reaktionsbedingungen gehandhabt wird. Dadurch sind eine erheblich vereinfachte Reaktionsführung und Reaktionskontrolle möglich.

Die bevorzugte Verfahrensführung der Partialhydrierung ist die kontinuierliche Fahrweise. Als Reaktoren sind beispielsweise gerührte oder durch Pumpen durchmischte Reaktoren bevorzugt, wobei das Einbringen von Wasserstoff möglichst effizient sein sollte. Dies kann z.B. durch Wellenbrecher in gerührten Systemen oder durch Stromstörer allgemein erreicht werden.

Gemäß einer unter anderem bevorzugten Ausführungsform der vorliegenden Erfindung wird dort, wo die Hydrierung stattfindet, gleichzeitig die freiwerdende Wärme abgeführt und damit beispielsweise Dampf erzeugt. Diese Verfahrensführung wird beispielsweise bevorzugt in mindestens einem Rohrbündelreaktor durchgeführt. Werden Rohrreaktorsysteme benutzt, so ist es vorteilhaft, beispielsweise durch geeignete Einbauten das Einmischen von Wasserstoff zu beschleunigen, wie es beispielsweise in gepackten Blasensäulen üblich ist.

Zur Vervollständigung des Umsatzes ist es im Rahmen der vorliegenden Erfindung möglich, mindestens zwei Reaktoren hintereinander zu betreiben. Dabei kann beispielsweise ein erster Reaktor stark durchmischt sein, was beispielsweise durch Produktrückführung mittels Pumpe erreicht werden kann, während ein zweiter und gegebenenfalls ein dritter Reaktor lediglich durchströmt werden, wobei gegebenenfalls Wasserstoff zugegeben werden kann. Gemäß einer bevorzugten Ausgestaltung dieser speziellen Verfahrensführung wird im ersten Reaktor ein Umsatz im Bereich von 80 bis 98 % erzielt, während der oder die Nachreaktoren den Restumsatz gewährleisten.

Beim Anfahren der Hydrierung ist es insbesondere bevorzugt, das Edukt Cyclododecatrien nicht oder nicht pur zusammen mit Katalysator und/oder Katalysatorprecursor vorzulegen, da es zu unerwünschten exothermen Reaktionen kommen kann. Im Allgemeinen kann mindestens ein geeignetes Lösungs- oder Verdünnungsmittel zugegeben werden. Als solche sind etwa Cyclododecan, Cyclododecen, gesättigte aliphatische Kohlenwasserstoffe, aromatische Kohlenwasserstoffe oder Gemische aus zwei oder mehr davon zu nennen. Gemäß einer bevorzugten Ausführungsform werden Cyclododecen oder Cyclododecan oder eine Mischung aus Cyclododecen und Cyclododecan oder eine Mischung aus Cyclododecen und Cyclododecatrien oder eine Mischung aus Cyclododecan und Cyclododecatrien oder eine Mischung aus Cyclododecen, Cyclododecan und Cyclododecatrien vorgelegt. Während der Gehalt der entsprechenden Mischungen an Cyclododecatrien im Allgemeinen unkritisch ist, liegt er in kontinuierlichen Verfahren vorzugsweise im Bereich von bis zu 30 Gew.-%, besonderes bevorzugt bei bis zu 25 Gew.-% und insbesondere bevorzugt bei bis zu 20 Gew.-%.

Demgemäß beschreibt die vorliegende Erfindung auch ein wie oben beschriebenes Verfahren, das dadurch gekennzeichnet ist, dass beim Anfahren der Partialhydrierung eine Mischung aus Cyclododecan und/oder Cyclododecen zusammen mit Cyclododecatrien vorgelegt wird, wobei der Gehalt dieser Mischung an Cyclododecatrien im Bereich von bis zu 30 Gew.-% liegt.

Das Produkt, das aus der erfindungsgemäßen Partialhydrierung erhalten wird, stellt im Allgemeinen ein Gemisch dar. Gemäß einer bevorzugten Ausführungsform enthält dieses Gemisch Cyclododecen in einem Bereich von 90 bis 99,9 Gew.-%, beispielsweise von 92 bis 99,9 Gew.-% oder im Bereich von 91 bis 99 Gew.-% weiter bevorzugt im Bereich von 92 bis 98 Gew.-%, besonders bevorzugt im Bereich von 94 bis 99 Gew.-% und insbesondere bevorzugt im Bereich von 96 bis 98 Gew.-%, jeweils bezogen auf das Gesamtgewicht des Produktgemisches.

Im Allgemeinen fällt das Cyclododecen als Gemisch aus cis- und trans-Isomer an. Im Allgemeinen liegt das Molverhältnis von cis-Isomer zu trans-Isomer im Bereich von 0,35:1 bis 2,0:1, bevorzugt im Bereich von 0,4:1 bis 2,0:1.

Neben Cyclododecen enthält das Produktgemisch im Allgemeinen Cyclododecan in einem Bereich von 0,1 bis 8 Gew.-%, bevorzugt in einem Bereich von 0,3 bis 7 Gew.-%, beispielsweise von 0,3 bis 5 Gew.% oder in einem Bereich von 0,5 bis 6,5 Gew.-% und besonders bevorzugt in einem Bereich von 0,5 bis 3 Gew.-%, jeweils bezogen auf das Gesamtgewicht des Produktgemisches.

Neben Cyclododecen und Cyclododecan kann das Produktgemisch Spuren von Cyclododecadienen und/oder nicht umgesetztem Cyclododecatrien und/oder Katalysator enthalten. Das erfindungsgemäße Verfahren kann prinzipiell so geführt werden, dass das eingesetzte Cyclododecatrien vollständig zu Cyclododecen umgesetzt wird. Im Allgemeinen enthält das Produktgemisch das nicht umgesetzte Edukt Cyclododecatrien in einer Menge von weniger als 0,5 Gew.-%, bevorzugt von weniger als 0,25 Gew.-% und insbesondere bevorzugt von weniger als 0,1 Gew.-%, Gew.-% und insbesondere bevorzugt von weniger als 0,1 Gew.-%, jeweils bezogen auf das Gesamtgewicht des Produktgemisches.

Sollte dies gewünscht sein, kann nicht umgesetztes Cyclododecatrien durch mindestens eine geeignete Methode wie beispielsweise bevorzugt mindestens eine destillative Maßnahme aus dem Produktgemisch abgetrennt und in das Verfahren rückgeführt werden. Besonders bevorzugt ist es im erfindungsgemäßen Verfahren, aufgrund des sehr hohen Umsatzes an Cyclododecatrien dieses aus dem Produktgemisch aus der Partialhydrierung nicht abzutrennen und Spuren von Cyclododecatrien zusammen mit dem Cyclododecen der Oxidierung mit Distickstoffmonoxid zuzuführen.

Gemäß einer bevorzugten Ausführungsform des erfindungsgemäßen Verfahrens wird der für die Partialhydrierung eingesetzte mindestens eine Katalysator aus dem Produktgemisch der Partialhydrierung abgetrennt. Diese Abtrennung kann in Abhängigkeit von eingesetztem Katalysator gemäß jeder geeigneten Verfahrensführung erfolgen.

Wird als Katalysator bei der Partialhydrierung beispielsweise ein heterogener Katalysator als Suspensionskatalysator eingesetzt, so wird dieser im Rahmen der vorliegenden Erfindung bevorzugt durch mindestens einen Filtrationsschritt abgetrennt. Der derart abgetrennte Katalysator kann im Folgenden entweder in das Verfahren rückgeführt werden oder in einem anderen Verfahrens eingesetzt werden, verworfen werden oder aufgearbeitet werden, beispielsweise, um das im Katalysator enthaltene mindestens eine Metall zurück zu gewinnen.

Wird als Katalysator bei der Partialhydrierung beispielsweise ein homogener Katalysator eingesetzt, so wird dieser im Rahmen der vorliegenden Erfindung bevorzugt durch mindestens einen Destillationsschritt abgetrennt. Im Rahmen dieser Destillation können eine oder zwei oder mehr Destillationskolonnen verwendet werden.

In der mindestens einen Destillationskolonne wird das Produktgemisch aus der Partialhydrierung in mindestens 2 Fraktionen aufgetrennt. Die Schwersiederfraktion enthält dabei im Wesentlichen die gesamte Menge des eingesetzten homogenen Hydrierkatalysators. Der derart abgetrennte Katalysator kann, gegebenenfalls nach mindestens einem geeigneten Regenerationsschritt, im Folgenden entweder in das Verfahren rückgeführt werden, verworfen werden oder aufgearbeitet werden, beispielsweise, um das im Katalysator enthaltene mindestens eine Metall zurückzugewinnen. Auch der Einsatz des abgetrennten Katalysators in einem anderen Verfahren ist möglich.

Gemäß einer besonders bevorzugten Ausführungsform des erfindungsgemäßen Verfahrens wird ein Teil des derart abgetrennten homogenen Hydrierkatalysators in das Verfahren rückgeführt und der Rest des abgetrennten Katalysators aus dem Verfahren rückgeführt und der Rest des abgetrennten Katalysators aus dem Verfahren ausgeschleust.

Die Hauptfraktion aus der obenstehend genannten destillativen Aufarbeitung des Produktgemisches aus der Partialhydrierung enthält im Wesentlichen Cyclododecen, mit kleinen Mengen Cyclododecan und gegebenenfalls Spuren an Cyclododecadienen, wie dies bereits obenstehend beschrieben ist.

Gemäß einer bevorzugten Ausführungsform des erfindungsgemäßen Verfahrens wird diese Hauptfraktion der Oxidation mit Distickstoffmonoxid zugeführt.

Ebenso ist es möglich, vor der Zuführung zur Oxidation in mindestens einem geeigneten Destillationsschritt Leichtsieder aus der Hauptfraktion abzutrennen.

Gemäß einer weiteren bevorzugten Ausführungsform des erfindungsgemäßen Verfahrens wird der für die Partialhydrierung eingesetzte mindestens eine Katalysator aus dem Produktgemisch der Partialhydrierung nicht abgetrennt. Diese Ausführungsform ist besonders bevorzugt, wenn für die Hydrierung ein homogener Katalysator eingesetzt wird. Weiter bevorzugt wird in diesem Fall das Produktgemisch aus der Partialhydrierung nicht aufgearbeitet und direkt der Oxidation mit Distickstoffmonoxid zugeführt.

Wie bereits obenstehend beschrieben, wird gemäß einer bevorzugten Ausführungsform im Rahmen der Oxidation des Cyclododecens mit Distickstoffmonoxid ein geeigneter Katalysator eingesetzt, der in der Lage ist, die Gleichgewichtseinstellung zwischen cis- und trans-Isomer des Cyclododecens zu katalysieren.

Gemäß einer besonders bevorzugten Ausführungsform wird als Katalysator für diese Gleichgewichtseinstellung der gleiche Katalysator verwendet wie für die Partialhydrierung des Cyclododecatriens.

Demgemäß betrifft die vorliegende Erfindung auch ein wie oben beschriebenes Verfahren, das dadurch gekennzeichnet ist, dass die Hydrierung von Cyclododecatrien zu Cyclododecen und die Umsetzung von Cyclododecen zu Cyclododecanon mit Distickstoffmonoxid in Anwesenheit des gleichen Katalysators erfolgen.

Einen erheblichen verfahrenstechnischen Vorteil des erfindungsgemäßen Verfahrens stellt die Tatsache dar, dass bei der Verwendung des gleichen homogenen Katalysators bei Partialhydrierung und Oxidation mit Distickstoffmonoxid der Katalysator aus dem Produktgemisch der Partialhydrierung nicht abgetrennt werden muss, sondern dieses Gemisch ohne aufwändige destillative Abtrennung des Katalysators direkt der Oxidation mit Distickstoffmonoxid zugeführt werden kann.

Demgemäß betrifft die vorliegende Erfindung auch ein wie oben beschriebenes Verfahren, das dadurch gekennzeichnet ist, dass ein aus der Hydrierung von Cyclododecatrien zu Cyclododecen in Anwesenheit eines homogenen Katalysators resultierendes Gemisch, enthaltend Cyclododecen und homogenen Katalysator, als Edukt für die Umsetzung mit Distickstoffmonoxid eingesetzt wird.

Im Rahmen der vorliegenden Erfindung ist es weiter möglich, aus dem Produktgemisch der Partialhydrierung nur einen Teil des Katalysators abzutrennen und das resultierende Gemisch, enthaltend Cyclododecen und den restlichen Teil des Katalysators, der Oxidation mit Distickstoffmonoxid zuzuführen. In diesem Fall kann gegebenenfalls mindestens ein weiterer Katalysator bei der Oxidation mit Distickstoffmonoxid zugesetzt werden. Weiter ist es möglich, den Katalysator aus dem Produktgemisch der Partialhydrierung nicht abzutrennen und bei der Oxidation mit Distickstoffmonoxid den gleichen und/oder mindestens einen weiteren Katalysator zuzusetzen.

Das oben beschriebene erfindungsgemäße Verfahren zur Herstellung von Cyclododecanon bietet unter anderem den Vorteil, dass Cyclododecanon in wenigen Schritten und gleichzeitig mit hoher Selektivität erhalten wird. Ein weiterer erheblicher Vorteil ist die Tatsache, dass als ein Edukt für das erfindungsgemäße Verfahren Distickstoffmonoxid enthaltende Abgase aus bevorzugt industriellen Anlagen eingesetzt werden die einerseits ohne großen Aufwand verfügbar sind, die andererseits die Integration des erfindungsgemäßen Verfahrens in einen bestehenden Anlagenverbund ermöglichen, wodurch der Transportweg für das Edukt minimal gehalten werden kann, und die weiterhin, als potentielle Klimagase, nicht einer besonderen Behandlung zur Entsorgung zugeführt werden müssen, sondern direkt in ein Wertprodukt fließen.

Das erfindungsgemäß hergestellte Cyclododecanon kann beispielsweise besonders bevorzugt zur Herstellung von Dodecandicarbonsäure und/oder Laurinlactam und/oder daraus abgeleiteten Polymeren wie beispielsweise Polyamiden wie Nylon 12 oder Nylon 6.12 eingesetzt werden.

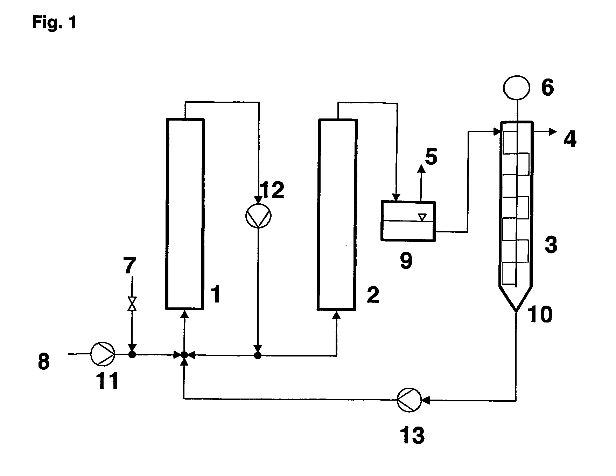

Die vorliegende Erfindung wird durch die nachfolgend beschriebene Figur 1 und die folgenden Beispiele illustriert.

Figur 1 beschreibt die im Rahmen der vorliegenden Erfindung unter anderem bevorzugte kontinuierliche Hydrierung von Cyclododecatrien zu Cyclododecen mittels eines homogenen Katalysators, wie sie beispielsweise in Beispiel 2 beschrieben ist. Dabei wird Cyclododecatrien-Edukt (8) über eine Pumpe (11) unter Beimengung von Wasserstoff (7) in einen ersten, kontinuierlich betriebenen Reaktor (1) geführt, wo ein erster Hydrierschritt stattfindet. Der Reaktionsaustrag aus dem Reaktor (1) wird über Pumpe (12) abgepumpt und gesplittet, wobei ein Teil des Reaktionsaustrags zur Verdünnung in den Reaktor (1) zurückgeführt wird und der andere Teil des Reaktionsaustrags als Feed in einen zweiten, ebenfalls kontinuierlich betriebenen Reaktor (2), den Nachreaktor, geführt wird. Der Reaktionsaustrag aus dem Reaktor (2) wird in einem Abscheider (9) in eine Flüssigphase und eine Gasphase getrennt und das Abgas (5) aus dem Abscheider (9) abgeführt. Nach Verlassen des Abscheiders (9) wird die Flüssigphase auf Umgebungsdruck entspannt und einem von einem Motor (6) angetriebenen Dünnschichtverdampfer (3) zugeführt. Aus der Abtrennung im Dünnschichtverdampfer (3) wird als Destillat das Produkt Cyclododecen (4) und als Sumpfprodukt (10) eine Flüssigphase erhalten, die über Pumpe (13) in den Reaktor (1) zurückgeführt wird. Diese Flüssigphase enthält den homogenen Hydrierkatalysator sowie einen Teil des Produkts (4) als Lösungsmittel für den Katalysator.

### Beispiele

### Beispiel 1 : Diskontinuierliche Hydrierung von Cyclododecatrien zu Cyclododecen

In einen 2,5 I Rührautoklaven wurden 1 kg trans,trans,cis-Cyclododeca-1,5,9-trien, 150 mg RuCl₃*H₂O, 20 g Triphenylphosphin, 12,5 g 37%iges wässriges Formaldehyd, 25 ml Ethanol und 2,5 g Essigsäure eingefüllt. Nach Spülen des Reaktors mit Stickstoff bzw. Wasserstoff wurden 15 bar Wasserstoff aufgepresst. Danach wurde der Reaktor unter Rühren aufgeheizt. Bei einer Temperatur von ca. 130 °C nahm der Reaktordruck merklich ab. Die Temperatur wurde anschließend auf 140 und 150 °C erhöht, der Druck wurde mittels Wasserstoffnachpressen auf 20 bar gehalten. Nach beendeter Wasserstoffaufnahme fanden sich im Reaktionsaustrag gemäß gaschromatographischer Analyse Cyclododecen mit 98,1 % und Cyclododecan mit 1,8 % Ausbeute.

### Beispiel 2 : Kontinuierliche Hydrierung von Cyclododecatrien zu Cyclododecen

1 kg Cyclododecen, 150 mg RuCl₃*H₂O, 20 g Triphenylphosphin, 12,5 g 37%iger wässriger Formaldehyd, 25 ml Ethanol und 2 g Adipinsäure wurden in den ersten Reaktor (Inhalt ca. 1 Liter) einer Versuchsapparatur gemäß Fig. 1 eingefüllt. Nach Aufheizen des Reaktorsystems auf 100 °C wurde die Umlaufpumpe in Betrieb genommen, mittels Wasserstoff der Druck auf 20 bar gebracht und ein Zulauf von 200 g Cyclododecatrien eingestellt. Die Reaktionstemperatur im ersten wie im zweiten Reaktor (Inhalt ca. 0,6 Liter) wurde auf ca. 140 °C eingestellt. Der Reaktionsaustrag Liter) wurde auf ca. 140°C eingestellt. Der Reaktionsaustrag wurde nach Entspannen auf Umgebungsdruck in einem Dünnschichtverdampfer so aufgetrennt, dass ca. 10 g/h Sumpfprodukt und 190 g/h Destillat anfielen. Das Sumpfprodukt wurde mittels Pumpe in den ersten Reaktor zurückgepumpt. Im Destillat fanden sich nach 24 h Betriebszeit ca. 97 % Cyclododecen, 2,6 % Cyclododecan sowie einige weitere, mengenmäßig unbedeutende Produkte.

### Beispiel 3 : Umsetzung von Cyclododecen mit N₂O

In einem 250 ml Autoklav wurden 0,5 mol Cyclododecen (als Gemisch mit 64% trans- und 33% cis-Anteil, Produkt aus Beispiel 2) vorgelegt. Der Autoklav wurde dann verschlossen und mit N₂ gespült. Anschließend wurde der Autoklav mit N₂O bis 50 bar aufgepresst. Die Temperatur wurde dann auf 250°C erhöht (maximaler Druck während der Reaktion: 84 bar). Nach 20 h Reaktionszeit wurde der Autoklav abgekühlt und entspannt. Der Autoklaveninhalt war bereits zum Teil kristallisiert. Um das Produkt zu analysieren, wurden der Inhalt bei 60°C aufgeschmolzen und eine homogene Probe entnommen. Nach Verdünnung mit Toluol wurde die Probe mittels quantitativer GC analysiert. Der Umsatz an trans-Cyclododecen betrug 98%. Der Umsatz an cis-Cyclododecen betrug 24%. Der Gesamtumsatz an Cyclododecen betrug 71 %. Die Selektivität zu Cyclododecanon lag bei mehr als 95 %. Als Nebenprodukte konnten mit GC/MS lediglich Spuren von Cyclododecenepoxid und 11-Dodecanal nachgewiesen werden.

### Beispiel 4 : Umsetzung von Cyclododecen mit N₂O

In einem 250 ml Autoklav wurden 0,52 mol Cyclododecen (als Gemisch mit 64% trans- und 33% cis-Anteil, Produkt aus Beispiel 2) vorgelegt. Der Autoklav wurde dann verschlossen und mit N₂ gespült. Anschließend wurde der Autoklav mit N₂O bis 50 bar aufgepresst. Die Temperatur wurde dann auf 275°C erhöht (maximaler Druck während der Reaktion: 122 bar). Nach 10 h Reaktionszeit wurde der Autoklav abgekühlt und entspannt. Der Autoklaveninhalt war bereits zum Teil kristallisiert. Um das Produkt zu analysieren, wurde der Inhalt bei 60°C aufgeschmolzen und eine homogene Probe entnommen. Nach Verdünnung mit Toluol wurde die Probe mittels quantitativer GC analysiert. Der Umsatz an trans-Cyclododecen betrug 99%. Der Umsatz an cis-Cyclododecen betrug 36%. Der Gesamtumsatz an Cyclododecen betrug 76%. Die Selektivität zu Cyclododecanon lag bei mehr als 95 %.

### Beispiel 5: Umsetzung von Cyclododecen mit N₂O ohne Abtrennung des Katalysators aus der Partialhydrierung

In einem 250 ml Autoklav wurden 0,5 mol Cyclododecen (Produktgemisch aus Beispiel 1, das noch Ru-Katalysator enthielt) vorgelegt. Der Autoklav wurde dann verschlossen und mit N₂ gespült. Anschließend wurde der Autoklav mit N₂O bis 50 bar aufgepresst. Die Temperatur wurde dann auf 250°C erhöht (maximaler Druck während der Reaktion: 79 bar). Nach 10 h Reaktionszeit wurde der Autoklav abgekühlt und entspannt. Der Autoklaveninhalt war bereits zum Teil kristallisiert. Um das Produkt zu analysieren, wurden der Inhalt bei 60°C aufgeschmolzen und eine homogene Probe entnommen. Nach Verdünnung mit Toluol wurde die Probe mittels quantitativer GC analysiert. Der Umsatz an trans-Cyclododecen betrug 75%. Der Umsatz an cis-Cyclododecen betrug 21 %. Die Selektivität zu Cyclododecanon lag bei mehr als 95 %.

### Beispiel 6: Umsetzung von Cyclododecen mit N₂O mit Abtrennung des Katalysators aus der Partialhydrierung

Beispiel 5 wurde wiederholt, wobei das Produktgemisch aus der Partialhydrierung, enthaltend Cyclododecen, zuerst durch Destillation vom Ru-Katalysator befreit wurde. Der Umsatz an trans-Cyclododecen betrug in diesem Fall 75%. Der Umsatz an cis-Cyclododecen lag jedoch bei weniger als 1 % (statt 21 % in Beispiel 5). Die Selektivität zu Cyclododecanon lag bei mehr als 95 %.

### Beispiel 7: Zweistufige Umsetzung von Cyclododecen mit N₂O ohne Abtrennung des Katalysators aus der Partialhydrierung

Das gemäß Beispiel 5 erhaltene Produkt wurde ohne weitere Behandlung wieder mit N₂O auf einen Enddruck von 50 bar bei Raumtemperatur aufgepresst, und das Gemisch wurde 20 Stunden bei 295°C gerührt (maximaler Druck während der Umsetzung: 245 bar). Anschließend wurde der Autoklav abgekühlt und entspannt. Um das Produkt zu analysieren, wurden der Inhalt bei 60°C aufgeschmolzen und eine homogene Probe entnommen. Nach Verdünnung mit Toluol wurde die Probe mittels quantitativer GC analysiert. Der Umsatz an trans-Cyclododecen betrug 99 %. Der Umsatz an cis-Cyclododecen betrug 32 %. Die Selektivität zu Cyclododecanon lag bei mehr als 95 %.

### Bezugszeichenliste

- 1: Reaktor 1
- 2: Reaktor 2
- 3: Dünnschichtverdampfer
- 4: Cyclododecen
- 5: Abgas
- 6: Antriebsmotor des Dünnschichtverdampfers
- 7: Wasserstoff
- 8: Cyclododecatrien
- 9: Abscheider
- 10: Sumpfprodukt
- 11: Pumpe
- 12: Pumpe
- 13: Pumpe

## Patentansprüche

1. Verfahren zur Herstellung von Cyclododecanon durch Umsetzung von Cyclododecen mit Distickstoffmonoxid, **dadurch gekennzeichnet, dass** als Distickstoffmonoxidquelle mindestens ein Distickstoffmonoxid enthaltendes Abgas mindestens eines industriellen Verfahrens dient.

2. Verfahren nach Anspruch 1, **dadurch gekennzeichnet, dass** die Distickstoffmonoxidquelle das Abgas einer Adipinsäureanlage und/oder einer Dodecandisäureanlage und/oder einer Hydroxylaminanlage und/oder einer mit dem Abgas einer Adipinsäureanlage und/oder einer Dodecandisäureantage und/oder einer Hydroxylaminanlage betriebenen Salpetersäureanlage ist.

3. Verfahren nach einem der Ansprüche 1 oder 2, **dadurch gekennzeichnet, dass** Cyclododecen mit einem Gasgemisch, enthaltend 20 bis 99,9 Gew.-% Distickstoffmonoxid, bezogen auf das Gesamtgewicht des Gasgemisches, umgesetzt wird.

4. Verfahren nach einem der Ansprüche 1 bis 3, **dadurch gekennzeichnet, dass** Distickstoffmonoxid oder das Gasgemisch enthaltend Distickstoffmonoxid in flüssiger Form eingesetzt wird.

5. Verfahren nach einem der Ansprüche 1 bis 4, **dadurch gekennzeichnet, dass** die Umsetzung kontinuierlich in mindestens einem Rohrreaktor bei einer Temperatur im Bereich von 140 bis 350 °C durchgeführt wird.

6. Verfahren nach einem der Ansprüche 1 bis 5, **dadurch gekennzeichnet, dass** ein Gemisch, enthaltend cis-Cyclododecen und trans-Cyclododecen, mit Distickstoffmonoxid in zwei Stufen umgesetzt wird.

7. Verfahren nach Anspruch 6, **dadurch gekennzeichnet, dass** in der ersten Stufe die Umsetzung bei einer Temperatur im Bereich von 140 bis 300 °C und in der zweiten Stufe die Umsetzung bei einer Temperatur im Bereich von 165 bis 325 °C durchgeführt wird, wobei die Temperatur in der ersten Stufe niedriger ist als die Temperatur in der zweiten Stufe.

8. Verfahren nach einem der Ansprüche 1 bis 7, **dadurch gekennzeichnet, dass** das Cyclododecen aus der katalytischen Hydrierung mindestens eines Cyclododecatriens erhalten wird.

9. Verfahren nach Anspruch 8, **dadurch gekennzeichnet, dass** die Hydrierung von Cyclododecatrien zu Cyclododecen und die Umsetzung von Cyclododecen zu Cyclododecanon mit Distickstoffmonoxid in Anwesenheit des gleichen Katalysators erfolgen.

10. Verfahren nach Anspruch 9, **dadurch gekennzeichnet, dass** ein aus der Hydrierung von Cyclododecatrien zu Cyclododecen in Anwesenheit eines homogenen Katalysators resultierendes Gemisch, enthaltend Cyclododecen und homogenen Katalysator, als Edukt für die Umsetzung mit Distickstoffmonoxid eingesetzt wird.

11. Verfahren zur Herstellung von Cyclododecanon, das die Schritte (I) und (II) umfasst:
(I) Herstellung von Cyclododecen durch Partialhydrferung von Cyclododecafien;
(II) Umsetzung von gemäß (I) erhaltenem Cyclododecen mit Distickstoffmonoxid unter Erhalt von Cyclododecanon,
**dadurch gekennzeichnet, dass** als Quelle des gemäß (II) eingesetzten Distickstoffmonoxids mindestens ein Distickstoffmonoxid enthaltendes Abgas mindestens eines industriellen Verfahrens dient.

12. Verfahren nach Anspruch 11, **dadurch gekennzeichnet, dass** die Distickstoffmonoxidquelle das Abgas einer Adipinsäureanlage und/oder einer Dodecandisäureanlage und/oder einer Hydroxylaminanlage und/oder einer mit dem Abgas einer Adipinsäureanlage und/oder einer Dodecandisäureanlage und/oder einer Hydroxylaminanlage betriebenen Salpetersäureanlage ist.

13. Verfahren nach einem der Ansprüche 11 oder 12, **dadurch gekennzeichnet, dass** Cyclododecen gemäß (II) mit einem Gasgemisch, enthaltend 20 bis 99,9 Gew.-% Distickstoffmonoxid, bezogen auf das Gesamtgewicht des Gasgemisches, umgesetzt wird.

14. Verfahren nach einem der Ansprüche 11 bis 13, **dadurch gekennzeichnet, dass** Distickstoffmonoxid oder das Gasgemisch enthaltend Distickstoffmonoxid in flüssiger Form eingesetzt wird.

15. Verfahren nach einem der Ansprüche 11 bis 14, **dadurch gekennzeichnet, dass** die Umsetzung gemäß (II) kontinuierlich in mindestens einem Rohrreaktor bei einer Temperatur im Bereich von 140 bis 350 °C durchgeführt wird.

16. Verfahren nach einem der Ansprüche 11 bis 15, **dadurch gekennzeichnet, dass** gemäß (II) ein Gemisch, enthaltend cis-Cyclododecen und trans-Cyclododecen, mit Distickstoffmonoxid in zwei Stufen umgesetzt wird.

17. Verfahren nach Anspruch 16, **dadurch gekennzeichnet, dass** in der ersten Stufe die Umsetzung bei einer Temperatur im Bereich von 140 bis 300°C und in der zweiten Stufe die Umsetzung bei einer Temperatur im Bereich von 165 bis 325 °C durchgeführt wird, wobei die Temperatur in der ersten Stufe niedriger ist als die Temperatur in der zweiten Stufe.

18. Verfahren nach einem der Ansprüche 11 bis 17, **dadurch gekennzeichnet, dass** die Partialhydrierung von Cyclododecatrien zu Cyclododecen gemäß (I) und die Umsetzung von Cyclododecen zu Cyclododecanon mit Distickstoffmonoxid gemäß (II) in Anwesenheit des gleichen Katalysators erfolgen.

19. Verfahren nach Anspruch 18, **dadurch gekennzeichnet, dass** ein aus der Partialhydrierung von Cyclododecatrien zu Cyclododecen in Anwesenheit eines homogenen Katalysators gemäß (I) resultierendes Gemisch, enthaltend Cyclododecen und homogenen Katalysator, als Edukt für die Umsetzung mit Distickstoffmonoxid gemäß (II) eingesetzt wird.

## Claims

1. A process for preparing cyclododecanone by reacting cyclododecene with dinitrogen monoxide, wherein the dinitrogen monoxide source is at least one dinitrogen monoxide-comprising offgas of at least one industrial process.

2. The process according to claim 1, wherein the dinitrogen monoxide source is the offgas of an adipic acid plant and/or of a dodecanedioic acid plant and/or of a hydroxylamine plant and/or of a nitric acid plant operated with the offgas of an adipic acid plant and/or of a dodecanedioic acid plant and/or of a hydroxylamine plant.

3. The process according to either of claims 1 and 2, wherein cyclododecene is reacted with a gas mixture comprising from 20 to 99.9% by weight of dinitrogen monoxide, based on the total weight of the gas mixture.

4. The process according to any of claims 1 to 3, wherein dinitrogen monoxide or the gas mixture comprising dinitrogen monoxide is used in liquid form.

5. The process according to any of claims 1 to 4, wherein the reaction is carried out continuously in at least one tubular reactor at a temperature in the range from 140 to 350°C.

6. The process according to any of claims 1 to 5, wherein a mixture comprising cis-cyclododecene and trans-cyclododecene is reacted with dinitrogen monoxide in two stages.

7. The process according to claim 6, wherein the reaction in the first stage is carried out at a temperature in the range from 140 to 300°C and the reaction in the second stage at a temperature in the range from 165 to 325°C, the temperature in the first stage being lower than the temperature in the second stage.

8. The process according to any of claims 1 to 7, wherein the cyclododecene is obtained from the catalytic hydrogenation of at least one cyclododecatriene.

9. The process according to claim 8, wherein cyclododecatriene is hydrogenated to cyclododecene and cyclododecene is converted to cyclododecanone using dinitrogen monoxide in the presence of the same catalyst.

10. The process according to claim 9, wherein the reactant used for the reaction with dinitrogen monoxide is a mixture which results from the hydrogenation of cyclododecatriene to cyclododecene in the presence of a homogeneous catalyst and comprises cyclododecene and homogeneous catalysts.

11. A process for preparing cyclododecanone, which comprises steps (I) and (II):
(I) preparing cyclododecene by partially hydrogenating cyclododecatriene;
(II) reacting cyclododecene obtained in (I) with dinitrogen monoxide to obtain cyclododecanone,
wherein the source used for the dinitrogen monoxide used in (II) is at least one offgas comprising dinitrogen monoxide from at least one industrial process.

12. The process according to claim 11, wherein the dinitrogen monoxide source is the offgas of an adipic acid plant and/or of a dodecanedioic acid plant and/or of a hydroxylamine plant and/or of a nitric acid plant operated with the offgas of an adipic acid plant and/or of a dodecanedioic acid plant and/or of a hydroxylamine plant.

13. The process according to either of claims 11 and 12, wherein cyclododecene is reacted in (II) with a gas mixture comprising from 20 to 99.9% by weight of dinitrogen monoxide, based on the total weight of the gas mixture.

14. The process according to any of claims 11 to 13, wherein dinitrogen monoxide or the gas mixture comprising dinitrogen monoxide is used in liquid form.

15. The process according to any of claims 11 to 14, wherein the reaction is carried out in (II) continuously in at least one tubular reactor at a temperature in the range from 140 to 350°C.

16. The process according to any of claims 11 to 15, wherein a mixture comprising cis-cyclododecene and trans-cyclododecene is reacted in (II) with dinitrogen monoxide in two stages.

17. The process according to claim 16, wherein the reaction in the first stage is carried out at a temperature in the range from 140 to 300°C and the reaction in the second stage at a temperature in the range from 165 to 325°C, the temperature in the first stage being lower than the temperature in the second stage.

18. The process according to any of claims 11 to 17, wherein cyclododecatriene is partially hydrogenated to cyclododecene in (I) and cyclododecene is converted to cyclododecanone using dinitrogen monoxide in (II) in the presence of the same catalyst.

19. The process according to claim 18, wherein the reactant used for the reaction with dinitrogen monoxide in (II) is a mixture which results from the partial hydrogenation of cyclododecatriene to cyclododecene in the presence of a homogeneous catalyst in (I) and comprises cyclododecene and homogeneous catalyst.

## Revendications

1. Procédé de préparation de cyclododécanone par réaction de cyclododécène avec du monoxyde de diazote, **caractérisé en ce qu'**au moins un effluent gazeux contenant du monoxyde de diazote d'au moins un procédé industriel sert de source de monoxyde de diazote.

2. Procédé selon la revendication 1, **caractérisé en ce que** la source de monoxyde de diazote est l'effluent gazeux d'une installation d'acide adipique et/ou d'une installation d'acide dodécanedioïque et/ou d'une installation d'hydroxylamine et/ou d'une installation d'acide nitrique fonctionnant avec l'effluent gazeux d'une installation d'acide adipique et/ou d'une installation d'acide dodécanedioïque et/ou d'une installation d'hydroxylamine.

3. Procédé selon l'une des revendications 1 ou 2, **caractérisé en ce que** le cyclododécène est amené à réagir avec un mélange gazeux, contenant 20 à 99,9 % en poids de monoxyde de diazote, par rapport au poids total du mélange gazeux.

4. Procédé selon l'une quelconque des revendications 1 à 3, **caractérisé en ce que** le monoxyde de diazote ou le mélange gazeux contenant du monoxyde de diazote est utilisé sous forme liquide.

5. Procédé selon l'une quelconque des revendications 1 à 4, **caractérisé en ce que** la réaction est mise en oeuvre en continu dans au moins un réacteur tubulaire à une température dans la plage de 140 à 350°C.

6. Procédé selon l'une quelconque des revendications 1 à 5, **caractérisé en ce qu'**un mélange, contenant du cis-cyclododécène et du trans-cyclododécène, est amené à réagir en deux étapes avec du monoxyde de diazote.

7. Procédé selon la revendication 6, **caractérisé en ce que** la réaction est mise en oeuvre dans la première étape à une température dans la plage de 140 à 300°C et la réaction est mise en oeuvre dans la deuxième étape à une température dans la plage de 165 à 325°C, la température dans la première étape étant inférieure à la température dans la deuxième étape.

8. Procédé selon l'une quelconque des revendications 1 à 7, **caractérisé en ce que** le cyclododécène est obtenu à partir de l'hydrogénation catalytique d'au moins un cyclododécatriène.

9. Procédé selon la revendication 8, **caractérisé en ce que** l'hydrogénation de cyclododécatriène pour donner du cyclododécène et la réaction de cyclododécène pour donner du cyclododécanone ont lieu avec du monoxyde de diazote en présence du même catalyseur.

10. Procédé selon la revendication 9, **caractérisé en ce qu'**un mélange résultant de l'hydrogénation de cyclododécatriène pour donner du cyclododécène en présence d'un catalyseur homogène, et contenant du cyclododécène et un catalyseur homogène, est utilisé en tant qu'éduit pour la réaction avec du monoxyde de diazote.

11. Procédé pour la préparation de cyclododécanone, comportant les étapes (I) et (II) consistant à :
(I) préparer du cyclododécène grâce à une hydrogénation partielle de cyclododécatriène ;
(II) amener le cyclododécène obtenu selon (I) à réagir avec du monoxyde de diazote pour obtenir du cyclododécanone,
**caractérisé en ce qu'**un effluent gazeux, contenant du monoxyde de diazote, d'au moins un procédé industriel sert de source du monoxyde de diazote utilisé en (II).

12. Procédé selon la revendication 11, **caractérisé en ce que** la source de monoxyde de diazote est l'effluent gazeux d'une installation d'acide adipique et/ou d'une installation d'acide dodécanedioïque et/ou d'une installation d'hydroxylamine et/ou d'une installation d'acide nitrique fonctionnant avec l'effluent gazeux d'une installation d'acide adipique et/ou d'une installation d'acide dodécanedioïque et/ou d'une installation d'hydroxylamine.

13. Procédé selon l'une des revendications 11 ou 12, **caractérisé en ce que** du cyclododécène selon (II) est amené à réagir avec un mélange gazeux contenant 20 à 99,9 % en poids de monoxyde de diazote, par rapport au poids total du mélange gazeux.

14. Procédé selon l'une quelconque des revendications 11 à 13, **caractérisé en ce que** le monoxyde de diazote ou le mélange gazeux contenant du monoxyde de diazote est utilisé sous forme liquide.

15. Procédé selon l'une quelconque des revendications 11 à 14, **caractérisé en ce que** la réaction est mise en oeuvre selon (II) en continu dans au moins un réacteur tubulaire à une température dans la plage de 140 à 350°C.

16. Procédé selon l'une quelconque des revendications 11 à 15, **caractérisé en ce qu'**un mélange, contenant du cis-cyclododécène et du trans-cyclododécène, est amené à réagir selon (II) en deux étapes avec du monoxyde de diazote.

17. Procédé selon la revendication 16, **caractérisé en ce que** la réaction est mise en oeuvre dans la première étape à une température dans la plage de 140 à 300°C et la réaction est mise en oeuvre dans la deuxième étape à une température dans la plage de 165 à 325°C, la température dans la première étape étant inférieure à la température dans la deuxième étape.

18. Procédé selon l'une quelconque des revendications 11 à 17, **caractérisé en ce que** l'hydrogénation partielle de cyclododécatriène pour donner du cyclododécène, selon (I), et la réaction du cyclododécène pour donner du cyclododécanone avec du monoxyde de diazote, selon (II), ont lieu en présence du même catalyseur.

19. Procédé selon la revendication 18, **caractérisé en ce qu'**un mélange résultant selon (I) de l'hydrogénation partielle de cyclododécatriène pour donner du cyclododécène en présence d'un catalyseur homogène, et contenant du cyclododécène et un catalyseur homogène, est utilisé selon (II) en tant qu'éduit pour la réaction avec du monoxyde de diazote.
